# EUROPEAN PATENT SPECIFICATION

(11) **EP 1 567 203 B1**
(45) Date of publication and mention of the grant of the patent: **26.09.2007**
(21) Application number: 03796727.0
(22) Date of filing: 05.12.2003
(51) Int. Cl.: A61L 29/08, A61L 29/16, A61L 31/10, A61L 31/16

(54) **BARRIERS FOR BIOACTIVE AGENT-CONTAINING POLYMERIC COATINGS**
SPERRSCHICHT FÜR WIRKSTOFFHALTIGE POLYMERE BESCHICHTUNGEN
BARRIERES POUR REVETEMENTS POLYMERIQUES

(30) Priority: 06.12.2002 US 313234
(43) Date of publication of application: 31.08.2005
(73) Proprietor: SurModics, Inc., Eden Prairie, MN 55344 (US)
(72) Inventor: LAWIN, Laurie, R., New Brighton, MN 55112 (US); BOUCHA-RAYLE, Michelle, C., Minnetonka, MN 55345 (US); KLOKE, Timothy, M., Chaska, MN 55318 (US)
(74) Representative: Gowshall, Jonathan Vallance
(86) International application number: PCT/US2003/038788
(87) International publication number: WO 2004/052420

(56) References cited:
- EP-A- 0 923 953
- WO-A-01/36008
- US-B1- 6 214 901

## Description

### TECHNICAL FIELD

In one aspect, the present invention relates to an implantable medical device having a surface providing an intact polymeric coating composition containing one or more bioactive agent(s) that provides release of the bioactive agent(s) from the surface of the device in vivo. In another aspect, the invention relates to methods and materials for protecting coated bioactive agent-containing compositions.

### BACKGROUND OF THE INVENTION

Many surgical interventions require the placement of a medical device, such as a catheter or stent, into the body. While necessary and beneficial for treating a variety of medical conditions, the placement of metal or polymeric devices in the body can give rise to numerous complications. Some of these complications include: increased risk of infection; initiation of a foreign body response resulting in inflammation and fibrous encapsulation; and initiation of a wound healing response resulting in hyperplasia and restenosis. These, and other complications are ideally dealt with prior to or upon introducing a metal or polymeric device into the body.

One approach to reducing the potential complications associated with such devices is to attempt to provide a more biocompatible implantable device. While there are several methods available to improve the biocompatibility of implantable devices, one method that has met with particular recent success is to provide the device with the ability to deliver bioactive compounds to the vicinity of the implant. By so doing, various potential drawbacks associated with the implantation of medical devices can be diminished. Thus, for example, antibiotics can be released from the surface of the device to minimize the possibility of infection, and anti-proliferative drugs can be released to inhibit hyperplasia. The ability to provide localized release of a bioactive agent in this manner lessens or avoids the need to deliver drugs systemically, or at localized but potentially problematic levels.

Although there are great potential benefits expected from the release of bioactive agents from the surfaces of medical devices, the development of such medical devices that can predictably and efficiently release bioactive agents after implantation has been slow. This development has been hampered by the many challenges that need to be successfully overcome when undertaking said development. Some of these challenges are: 1) the requirement for controlled and/or predictable, and in some instances for long term, release of bioactive agents; 2) the need for a biocompatible, non-inflammatory device surface; 3) the need for significant tenacity and durability, particularly for coatings upon devices that undergo flexion and/or expansion when being implanted or used in the body; 4) concerns regarding the ability to fabricate such device/bioactive agent combinations, to enable the device to be manufactured in an economically viable and reproducible manner; 5) the requirement that the device either be fabricated in a sterile manner, or the finished device be capable of being sterilized using conventional methods; and 6) the requirement that a coating (e.g., as provided by a polymeric coating composition containing a bioactive agent) needs to remain intact and undamaged during and after insertion in the course of a surgical procedure.

Several implantable medical devices capable of delivering bioactive agents have been described. Several patents are directed to devices utilizing biodegradable or bioresorbable polymers as drug containing and releasing coatings, including Tang et al, U.S. Patent 4,916,193 and MacGregor, U.S. Patent 4,994,071. Other patents are directed to the formation of a drug containing hydrogel on the surface of an implantable medical device, these include Amiden et al, U.S. patent 5,221,698 and Sahatjian, U.S. Patent 5,304,121. Still other patents describe methods for preparing coated intravascular stents via application of polymer solutions containing dispersed therapeutic material to the stent surface followed by evaporation of the solvent. This method is described in Berg et al, U.S. Patent 5,464,650.

Various other references relate to the use of coatings to provide implantable medical devices with bioactive agents. See, for instance, US 20020007213, and published PCT Application Nos. WO 200187372, WO 200187373, WO 200187374, WO 200187375, WO 200187376, WO 200226139, WO 200226271, WO 200226281, WO 200187342, and WO 200187263.

Included within these teachings is published US application No. 2002/0111590 , which describes implantable medical devices, such as stents, that are provided with polymeric coatings having therapeutic drugs, agents, or compounds. Pages 19-20, in particular, refer to various problems relating to the removal of drug, agent, or compound coating during delivery of a coated device such as a stent, such as by retraction of a restraining sheath, or by expansion of a balloon within the stent. The section refers, without apparent supporting examples, to a textbook type array of "lubricious coatings" that might be used to solve this problem, particularly including the use of silicone, synthetic waxes, natural products, fluorinated compounds, synthetic polymers, and inorganic materials. Included within the synthetic polymers are "silicones e.g. polydimethylsiloxane, polytetrafluoroethylene, polyfluoroethers, polyalkylglycol e.g. polyethylene glycol waxes". With regard to the particular stent design described therein, the application describes (at paragraph 0187) a "related" problem in which the movement of various stent portions, in the course of expansion, leads to further degradation of the coating, in response to which application suggests the use of water soluble powders.

Applicants have themselves previously described an implantable medical device that can undergo flexion and/or expansion upon implantation, and that is also capable of delivering a therapeutically significant amount of a bioactive agent or agents from the surface of the device. Applicants' issued US Patent No. 6,214,901 and published PCT Application No. WO 00/55396 provide such coating compositions, including those that comprises at least one polyalkyl(meth)acrylate, as a first polymeric component and poly(ethylene-co-vinyl acetate) ("pEVA") as a second polymeric component, and describe the use of such compositions for coating an implant surface using any suitable means, e.g., by dipping, spraying and the like. Coatings such as those described and claimed by Applicants are referred to and described as preferred in the '590 application discussed above.

In spite of the description of the '590 application, there clearly remains a need for methods or materials that will minimize or avoid damage to or delamination of bioactive agent-containing coatings upon medical devices. This is particularly true for medical devices, such as stents, that undergo flexion and tortuous movement in the course of their preparation, deployment and use. It is even more true for bioactive agent-containing coatings in which the agent concentration is particularly high, sometimes as high as 30% by weight or more, or even 40% or more, of the total weight of the coated compositions. While higher agent loading is generally desired, given the generally small surface areas involved and in order to deliver more bioactive agent to the particular site, increasing amounts of such agents can have the tendency to weaken the integrity of the coating itself, exacerbating concerns of damage or delamination.

On yet another topic, various references describe the use of polymeric coatings on coated or uncoated devices, albeit without the inclusion ofbioactive agents, though for a variety of purposes, see, for example, US Patent Nos. 5,569,463 (Helmus, et al.); 5,674,241 (Bley, et al.); 6,251,136 (Guruwaiya, et al.); 6,287,285 (Michal, et al.); 6,451,373 (Hossainy, et al.), Published International Application No. WO 9938546 (Michal, et al.), and published US Application Nos. US20020054900A1 (Kamath, et al.), US 20020055721A1 (Palasis, et al.) and US20020138048A1 (Tuch).

Document D1 discloses (cf. col. 3, par. 13 - col. 4, par. 9 ; claims 1, 2, 9) implantable medical devices with a first polymeric coating containing a biologically active material and a second polymeric coating of fluorosilicone or polyethylene glycol.

On separate subjects, tri-block polymers such as those known commonly as poloxamers have themselves been used as drug releasing matricies. See, for example, MR Kim, et al., "Temperature-responsive and degradable hyaluronic acid/Pluronic composite hydrogels for controlled release of human growth hormone", J. Control Release 2002 Apr 23;80(1-3):69-77.

Also, Applicants' own previous patents and applications describe an array of polymers having one or more attached latent reactive groups, such as photoreactive groups, that permit the polymers to be attached to various surfaces, and/or other molecules, in order to achieve a corresponding array of purposes. On yet another subject, the assignee of the present invention has previously described a variety of applications for the use of photochemistry, and in particular, photoreactive groups, e.g., for attaching polymers and other molecules to support surfaces. See, for instance, US Patent Nos. 4,722,906, 4,826,759, 4,973,493, 4,979,959, 5,002,582, 5,217,492, 5,258,041, 5,263,992, 5,414,075, 5,512,329, 5,563,056, 5,637,460, 5,714,360, 5,741,551, 5,744,515, 5,783,502, 5,858,653, 5,942,555, 5,981,298, 6,007,833, 6,077,698, 6,090,995, 6,121,027, 6,156,345, and published PCT Application Nos. WO8802623, WO9000887, W08905616, W09103990, W09316176, W09411032, WO9637165, WO9639821, WO9716544, WO9734935, WO9908717, WO9916907, WO9943688, WO9947129, WO9947176, WO9964086, W00121326, WO0040593, WO01441 and WO01661.

To the best of Applicants' knowledge, however, no reference presently describes and enables the use of a barrier for the purpose of preventing damage to and/or delamination of a polymeric coating by contacting other, coated or uncoated, surfaces, and particular with coatings that contain high concentrations of bioactive agents and that are positioned upon devices that undergo flexion in the course of their deployment or use.

### SUNINIARY OF THE INVENTION

The present invention provides a barrier as defined in the claims, e.g., in the form of a discrete anti-adherent film or coating composition, adapted to be positioned between a first surface provided in the form of a polymeric, bioactive agent-containing coating upon a medical device, and a second surface provided by another material positioned in apposition, and preferably moveable apposition, to the first surface. By "moveable apposition" it is meant that the two surfaces are moved in the course of their manufacture or use, e.g., abraded, bent, or expanded with respect to each other.

The barrier comprises a polymer selected from the group consisting of block copolymers and polymers bearing latent reactive groups. The former are particularly useful in view of their ability to provide regions of discrete properties, such as hydrophobicity, which can be adapted to what are typically very different surface characteristics as between a polymer coated surface and a different material such as a balloon. The latter are particularly useful in view of their ability to be manufactured and designed to provide particular physico-chemical properties, and to then be covalently bound in a desired manner (e.g., to the first and/or second surfaces, or there between), upon activation of the latent reactive groups.

Preferred block copolymers of the present invention are ethylene oxide/propylene oxide block copolymers, and particularly those water-soluble, diblock and triblock copolymers know as poloxamers, such as those available as surfactants under the tradenames Pluronic, Lutrol and Tetronic, each available from BASF Corp., Mt. Olive, NJ. Such copolymers can be provided with an optimal combination of amorphous and crystallizable blocks.

Particularly preferred polymers for the present invention are those available as PLURONIC (TM) F-127 and F-108. These viscosity modifiers are block copolymers of ethylene oxide and propylene oxide. Thickening tendencies of block copolymers increase as ethylene oxide content and total molecular weight increase. Thermally responsive block copolymers have been disclosed in U. S. Pat. Nos. 4,474,751; 4,474,752; 5,441,732; and 5,252,318, as well as the Product Catalog, "BASF Performance Chemicals. These block copolymers offer extremely low toxicity and a high degree of mildness for applications involving human contact.

For the preparation of photoderivatized polymers of this invention, preferred latent reactive polymers are those that include, as one or more latent reactive groups, the use of photoreactive groups such as aryl ketones, and more particularly, benzophenone. The polymers themselves can be either natural or synthetic in nature. Preferred natural polymers include polysaccharides, including hyaluronic acid and mucopolysaccharides such as heparin, and polypeptides (including proteins).

Preferred synthetic polymers, for instance, are photoderivatized polyolefins (e.g., polyethylenes, polypropylenes, polybut-1-enes, polyisobutylenes, diene rubbers, cyclo-olefins, and 1,2-polybutadienes), vinyl chloride polymers, fluorine-containing polymers (e.g., polytetrafluoroethylenes), poly(vinyl acetates), poly(vinyl alcohols), poly(vinyl acetals), polyacrylates and polymethacrylates, styrene polymers and copolymers, vinyl thermoplastics, polyamides and polyimides, polyacetals, polycarbonates, thermoplastics containing p-phenylene groups (e.g., polyphenylenes, polysulphones), polyesters, polyurethanes, polyisocyanurates, and silicones, including copolymers and blends of each.

Particularly preferred are photoderivatized amides, such as photoderivatized polyacrylamide copolymers, and photoderivatized vinyl thermoplastics, such as photopolyvinylpyrrolidone copolymers. The preparation of such photoderivatized polymers can be achieved in any suitable manner, as by copolymerizing monomers with monomers containing photoreactive groups, or by derivatizing a formed polymer with such photogroups, as by the use of corresponding photoreagents. An example of the preparation of a photoderivatized polyacrylamide can be found, for instance, in at Example 2 of Applicants' European Application No. 585436. Photopolyvinylpyrrolidone ("photoPVP") is also available commercially, e.g., under the product name "PV05", from Surmodics, Inc., Eden Prairie, MN, or can be synthesized as well. Synthesis ofphotoPVP can be accomplished, for instance, by the free radical polymerization of 1-vinyl-2-pyrrolidone monomers with photomonomers. Exemplary photomonomers, in turn, are described in U.S. Pat. No. 5,002,582.

Photoderivatized polysaccharides such as heparin ("photoheparin") can be prepared by those skilled in the art as well, e.g., in the manner described at Example 4 of US Patent.No. 5,563,056, which describes the preparation of photoheparin by reacting heparin with benzoyl-benzoyl-epsilon-aminocaproyl-N-oxysuccinimide in dimethylsulfoxide/carbonate buffer. The solvent was evaporated and the photoheparin was dialyzed against water, lyophilized, and then dissolved in water.

A barrier of this invention is adapted to prevent the second surface from damaging and/or delaminating the polymeric coating upon the first surface, either in the course of fabrication, storage, delivery or deployment, and/or residence of the device within the body. The barrier is adapted to prevent damage to and/or delamination of the polymeric coating in the course of whatever contact or relative movement may be encountered between the polymeric surface and the second surface. The barrier can be used in a manner analogous to the use of slip agents, generally provided as polymeric films positioned between other films or between films and production equipment in order to minimize friction or adherence between the various surfaces.

In turn, the barrier provides either continuous or discontinuous physical separation between the first and second surfaces, in a manner sufficient to prevent or lessen their direct contact, and in turn to prevent their adherence to each other. In addition to physical separation, the barrier preferably also provides an optimal combination of such properties as physico-chemical compatibility with the first and second surfaces, respectively, biocompatibility within the body, negligible or manageable interactions with bioactive release kinetics, and the ability to remain in the desired position, with respect to either the first and/or second surfaces, per se, or there between.

The barrier can be provided in the form of a permanent, removable, or transient (e.g., sacrificial) coating upon the polymeric coating and/or upon the second surface, and/or as a discrete layer positioned between the two. The barrier can itself be comprised of one or more layers, e.g., of the same or different materials, and positioned in any suitable combination upon the first and/or second surfaces, or separately provided between the two.

The barrier is particularly preferred for use with bioactive agent-containing polymeric coatings in which the agent is present at a concentration of at least 20%, more preferably 30%, and most preferably 40% by weight, based on the weight of the coated composition. In this manner, the use of the barrier can serve to counter the lack of structural integrity or elasticity imposed on the polymer coating, due at least in part to high agent loading.

In a further embodiment, the invention provides a combination comprising an implantable medical device comprising a surface having positioned thereon a polymeric coating, a barrier, and the surface of another material positioned in apposition to the barrier, and in turn, to the polymeric coating. In yet a further embodiment, the invention provides a method of making and a method of using the barrier, as well as combinations of the barrier with the coated medical device surface and/or the second surface.

In a particularly preferred example, for instance, the polymeric, bioactive agent-containing coating is positioned upon the surface of an implantable medical device, the second surface is provided by the surface of a different material (e.g., external delivery sheath or internal balloon) in apposition to the device, and the barrier is provided in the form of an anti-adherent coating adapted to facilitate the positioning of the medical device surface and the different material(s) in stable, and preferably separable, apposition to each other.

### DETAILED DESCRIPTION OF THE INVENTION

The term "coating composition", as used herein with respect to formation of a polymeric, bioactive agent-containing coating, will refer to one or more vehicles (e.g., a system of solutions, mixtures, emulsions, dispersions, blends etc.) used to effectively coat that surface with bioactive agent. The coating composition can include one or more polymer components, either individually or in any suitable combination (e.g., blend). In turn, the term "coated composition" will refer to the effective combination, upon a surface, of bioactive agent, and one or more polymer components (e.g., a combination of first polymer component and second polymer component), whether formed as the result of one or more coating vehicles, or in one or more layers.

Preferred polymer coatings provide a variety of common features, in that they tend to be hydrophobic, nonswellable, stable, biocompatible, adherent to the surface of the medical device, while also elastic and ductile, permitting the devices to be flexed and moved with the coatings remaining bound and/or clad thereto. Preferred barriers for use with such polymer coatings provide a corresponding array of features, including the ability to be retained by the polymer coating (as by the attraction of portions of a block polymer or activation of latent reactive groups), and in turn, to provide both spacing and lubricity with respect to a second material surface.

In one embodiment the polymeric coated composition, containing bioactive agent(s), can comprise at least one polyalkyl(meth)acrylate or polyaryl(meth)acrylate, as a first polymeric component, and poly(ethylene-co-vinyl acetate) ("pEVA") as a second polymeric component. A particularly preferred polymer mixture for use in this invention includes mixtures of poly(n-butyl methacrylate) ("pBMA") and poly(ethylene-*co*-vinyl acetate) co-polymers (pEVA). This mixture of polymers has proven useful with absolute polymer concentrations (i.e., the total combined concentrations of both polymers in the coating composition), of between about 0.05 and about 70 percent (by weight of the coating composition). In one preferred embodiment the polymer mixture includes a polyalkyl(meth)acrylate (such as poly(n-butyl methacrylate)) with a weight average molecular weight of from about 100 kilodaltons to about 1000 kilodaltons and a pEVA copolymer with a vinyl acetate content of from about 20 to about 40 weight percent.

In another embodiment the polymer mixture includes a polyalkyl(meth)acrylate (e.g., poly(n-butyl methacrylate)) with a molecular weight of from about 200 kilodaltons to about 500 kilodaltons and a pEVA copolymer with a vinyl acetate content of from about 30 to about 34 weight percent. The concentration of the bioactive agent or agents dissolved or suspended in the coating mixture can range from about 0.01 to about 90 percent, by weight, based on the weight of the final coating composition. Coating compositions comprising aromatic poly(meth)acrylates as described in Applicants' pending application USSN 10/174,635, filed June 18, 2002.

Suitable polymers, and bioactive agents, for use in preparing the polymeric, bioactive agent-containing coating compositions can be prepared using conventional organic synthetic procedures and/or are commercially available from a variety of sources, including for instance, from Sigma Aldrich (e.g., poly(ethylene-co-vinylacetate), and Polysciences, Inc, Warrington, PA (e.g., polybenzylmethacryate and poly(methyl methacrylate-co-n-butyl methacrylate). Optionally, and preferably, such polymer components are either provided in a form suitable for *in vivo* use, or are purified for such use to a desired extent (e.g., by removing impurities) by conventional methods available to those skilled in the art.

With regard to the bioactive agent-containing composition, examples of suitable first polymers for the coating composition include polyaryl(meth)acrylates, polyaralkyl(meth)acrylates, and polyaryloxyalkyl(meth)acrylates, in particular those with aryl groups having from 6 to 16 carbon atoms and with weight average molecular weights from about 50 to about 900 kilodaltons. Examples of polyaryl(meth)acrylates include poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate. Examples of polyaralkyl(meth)acrylates include polybenzylacrylate and methacrylate, poly-2-phenethylacrylate and methacrylate, poly-1-pyrenylmethylmethacrylate. Examples of polyaryloxyalkyl(meth)acrylates include polyphenoxyethylacrylate and methacrylate, polyethyleneglycolphenylether acrylates and methacrylates with varying polyethyleneglycol molecular weights.

A second polymer component for use in the bioactive agent-containing composition provides an optimal combination of similar properties, and particularly when used in admixture with the first polymer component. Examples of suitable second polymers are available commercially and include poly(ethylene-co-vinyl acetate) having vinyl acetate concentrations of between about 8% and about 90%, in the form of beads, pellets, granules, etc. pEVA co-polymers with lower percent vinyl acetate become increasingly insoluble in typical solvents.

A particularly preferred coating composition includes mixtures of polyalkyl(meth)acrylates (e.g., polybutyl(meth)acrylate) or aromatic poly(meth)acrylates (e.g., polybenzyl(meth)acrylate) and poly(ethylene-*co*-vinyl acetate) co-polymers (pEVA). This mixture of polymers has proven useful with absolute polymer concentrations (i.e., the total combined concentrations of both polymers in the coating composition), of between about 0.05 and about 70 percent (by weight), and more preferably between about 0.25 and about 10 percent (by weight). In one preferred embodiment the polymer mixture includes a first polymeric component with a weight average molecular weight of from about 100 kilodaltons to about 500 kilodaltons and a pEVA copolymer with a vinyl acetate content of from about 8 to about 90 weight percent, and more preferably between about 20 to about 40 weight percent. In a particularly preferred embodiment the polymer mixture includes a first polymeric component with a molecular weight of from about 200 kilodaltons to about 400 kilodaltons and a pEVA copolymer with a vinyl acetate content of from about 30 to about 34 weight percent. The concentration of the bioactive agent or agents dissolved or suspended in the coating mixture can range from about 0.01 to about 90 percent, by weight, based on the weight of the final coating composition.

The present invention provides a barrier, preferably in the form of an anti-adherent film or coating composition, and related method for using such a barrier upon or in apposition to a surface. By "anti-adherent", as used herein, it is meant that the barrier can be placed in apposition to the coating composition and/or other material under conditions that permit the coating composition and other material to be used (e.g., separated) without undue damage to the surface of either (of a type otherwise caused by the "adherence" of one to the other). The barrier may itself be positioned upon (e.g., stably coated upon) either surface, or adhered to neither surface, and instead be freely moveable between the two.

In turn, the barrier permits the coated surface of the medical device to be implanted *in vivo,* in a manner that protects the coated polymeric composition from mechanical damage and/or delamination, and enables the bioactive agent(s) to be predictably released over time. Preferred barriers are compatible with the coated composition, such that they either do not detrimentally affect the desired release of bioactive agent from the coating, or they affect that release in a desired or predictable manner.

In a further preferred embodiment, the barrier is provided in the form of an anti-adherent coating composition selected from the group consisting of block copolymers and polymers bearing latent reactive groups, and is adapted to be applied to and retained upon a coated bioactive agent-containing composition.

Both the polymeric coating and barrier (e.g., anti-adherent coating composition) can be provided in any suitable form, e.g., in the form of a film, a true solution, a fluid or paste-like emulsion, a mixture, a dispersion or a blend. In turn, and particularly where the barrier is itself coated, the coated barrier will generally result from the removal of solvents or other volatile components and/or other physical-chemical actions (e.g., heating or illuminating) affecting the coated composition *in situ* upon the surface.

A barrier of this invention will provide an optimal combination of properties between the barrier and the polymer coated surface (including any effects on bioactive agent release kinetics), the barrier and the contacting device surface, biocompatibility, physical and chemical stability. With regard to bioactive agent release kinetics, the barrier is preferably inert in this respect, or provides an impact that can be anticipated and factored into the preparation of the polymer coating itself, in order to achieve a desired net result. The release layer of this invention is preferably biocompatible, e.g., such that it results in no induction of inflammation or irritation when implanted. In addition, and particularly where the layer is itself provided by a plurality of polymer components, the composition is preferably useful under a broad spectrum of both absolute and relative polymer concentrations. This means that the physical characteristics of the layer or coating, such as tenacity, durability, flexibility and expandability, will typically be adequate over a broad range of polymer concentrations. The barrier is preferably provided without bioactive agent, but optionally can include the same or different bioactive agents as the underlying coated surface itself, or can include various other adjuvants. Other adjuvants such as polymerization catalysts, medicaments, indicators, dyes, wetting agents, buffering agents, thixotropes and the like can be included in the "barrier", contingent upon attainment of the desired degree of "protection" performance and suitability for use.

Devices useful in the present invention include medical devices, and preferably those that undergoes flexion and/or expansion in the course of implantation or use *in vivo.* In a particularly preferred embodiment, the present invention relates to a barrier (e.g., anti-adherent coating composition) and related method for coating an implantable medical device which undergoes flexion and/or expansion upon implantation with an anti-adherent coating composition. The structure and composition of the underlying device can be of any suitable, and medically acceptable, design and can be made of any suitable material that is compatible with the coating itself. The surface of the medical device is provided with a coating containing one or more bioactive agents.

The barrier provides the ability to deliver bioactive agents from undamaged coated polymeric compositions positioned upon devices that can themselves be fabricated from a variety of biomaterials. Preferred biomaterials include those formed of synthetic polymers, including oligomers, homopolymers, and copolymers resulting from either addition or condensation polymerizations. Examples of suitable addition polymers include, but are not limited to, acrylics such as those polymerized from methyl acrylate, methyl methacrylate, hydroxyethyl methacrylate, hydroxyethyl acrylate, acrylic acid, methacrylic acid, glyceryl acrylate, glyceryl methacrylate, methacrylamide, and acrylamide; vinyls such as ethylene, propylene, styrene, vinyl chloride, vinyl acetate, vinyl pyrrolidone, vinylidene difluoride, and fluorinated olefins (such as hexafluoropropylene). Examples of condensation polymers include, but are not limited to, nylons such as polycaprolactam, polylauryl lactam, polyhexamethylene adipamide, and polyhexamethylene dodecanediamide, and also polyurethanes, polycarbonates, polyamides, polysulfones, poly(ethylene terephthalate), polylactic acid, polyglycolic acid, polydimethylsiloxanes, and polyetheretherketone.

Certain natural materials are also suitable biomaterials, including human tissue such as bone, cartilage, skin and teeth; and other organic materials such as wood, cellulose, compressed carbon, and rubber. Other suitable biomaterials include metals and ceramics. The metals include, but are not limited to, titanium, stainless steel, and cobalt chromium. A second class of metals include the noble metals such as gold, silver, copper, and platinum. Alloys of metals, such as nitinol, may be suitable for biomaterials as well. The ceramics include, but are not limited to, silicon nitride, silicon carbide, zirconia, and alumina, as well as glass, silica, and sapphire. Combinations of ceramics and metals would be another class of biomaterials. Another class of biomaterials are fibrous or porous in nature. The surface of such biomaterials can be pretreated (e.g., with a Parylene coating composition) in order to alter the surface properties of the biomaterial.

Biomaterials can be used to fabricate a variety of implantable devices. General classes of suitable implantable devices include, but are not limited to, vascular devices such as grafts, stents, catheters, valves, artificial hearts, and heart assist devices; orthopedic devices such as joint implants, fracture repair devices, and artificial tendons; dental devices such as dental implants and fracture repair devices; drug delivery devices; ophthalmic devices and glaucoma drain shunts; urological devices such as penile, sphincter, urethral, bladder, and renal devices; and other catheters, synthetic prostheses such as breast prostheses and artificial organs. Other suitable biomedical devices include dialysis tubing and membranes, blood oxygenator tubing and membranes, blood bags, sutures, membranes, cell culture devices, chromatographic support materials, biosensors, and the like.

The surface contacting the polymer-coated medical device can be provided by any suitable means, e.g., as another surface of the same device, as a surrounding sheath or cover, or as an internal or contained material such as a balloon. Balloons, in turn, can be fabricated from a variety of materials, including for instance, polyethylene terephthalate, polyethylene, polyurethane, latex and nylon.

The present invention therefore provides a facile and easily processable method of ensuring the controlled and/or predictable rate of bioactive release from the surface of the device. Anti-adherent coating compositions applied over the polymeric coated composition provide a means to ensure that the composition remains intact and performs in the designed manner. A barrier, and particularly an anti-adherent coating composition, can be applied at any suitable time, e.g., before, during or after fabrication of the first or second surfaces, or their placement in apposition to each other. In a particularly preferred embodiment, an anti-adherent coating is applied after a polymeric coating composition, containing bioactive agent(s) or to which bioactive agent(s) have been applied, has been coated upon a first surface provided by the medical device.

A preferred barrier of this invention is provided as an anti-adherent coating composition adapted to be applied directly or indirectly to the surface of a coated polymeric composition, including a composition that itself contains bioactive agent(s), on an implantable medical device which undergoes flexion and/or expansion upon implantation or use. The anti-adherent coating composition may optionally be cured (e.g., solvent evaporated) to provide a suitably flexible and protective coating composition on the surface of the polymeric composition on the surface of the medical device. The anti-adherent coating composition provides protection to the polymeric composition from mechanical damage and/or delamination during the insertion of the medical device.

An anti-adherent coating composition, for use as a barrier, can be applied to the coated polymeric composition on the device in any suitable fashion, e.g., it can be provided in the form of a discrete film, or it can be applied as a coating composition directly to the surface of the coated polymeric composition on the medical device by methods that include airbrushing, atomized spraying, ultrasonic spraying, dipping, spray drying, vacuum deposition, electrostatic deposition, mechanical deposition, and lyophilizing. The method of applying the coating composition to the device is typically governed by the geometry of the device and other process considerations.

The bioactive agents useful in the present invention include virtually any therapeutic substance which possesses desirable therapeutic characteristics for application to the implant site. These agents include: thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti-polymerases, antiviral agents, photodynamic therapy agents, and gene therapy agents.

An anti-adherent coating composition for use as a barrier of this invention can be used to coat the polymeric composition upon the surface of a variety of devices, and is particularly useful for those devices that will come in contact with aqueous systems. Such devices are coated with a polymeric coating composition containing one or more bioactive agents, such that the coated composition is adapted to release the bioactive agent(s) in a controlled and/or predictable manner, generally beginning with the initial contact between the device surface and its aqueous environment.

An coating composition of this invention is preferably used to coat a polymeric coating composition on an implantable medical device that undergoes flexion or expansion in the course of its implantation or use in vivo. The words "flexion" and "expansion" as used herein with regard to implantable devices will refer to a device, or portion thereof, that is bent (e.g., by at least 45 degrees or more) and/or expanded (e.g., to more than twice its initial dimension), either in the course of its placement, or thereafter in the course of its use *in vivo.*

Examples of suitable catheters include urinary catheters, which would benefit from the incorporation of antimicrobial agents (e.g., antibiotics such as vancomycin or norfloxacin) into a surface coating, and intravenous catheters which would benefit from antimicrobial agents and or from antithrombotic agents (e.g., heparin, hirudin, coumadin). Such catheters are typically fabricated from such materials as silicone rubber, polyurethane, latex and polyvinylchloride.

A barrier coating composition overcoating the polymeric coating composition containing bioactive agent(s) is useful to coat stents, e.g., either self-expanding stents, which are typically prepared from nitinol, or balloon-expandable stents, which are typically prepared from stainless steel. Other stent materials, such as cobalt chromium alloys, can be coated by the coating composition as well.

The relative and overall thicknesses or weights of the various layers, including bioactive agent-containing polymeric layer(s), other polymeric layers, and/or the barrier itself upon the surface is typically not critical, so long as they collectively provide the desired release and compatability.

It is expected that the barrier need not add appreciably to the weight or thickness of the composite coating upon the surface of a medical device, hence the values described by Applicants previously remain applicable. In turn, the final coating thickness of a presently preferred combined barrier and polymeric coated composition will typically be in the range of about 0.1 micrometers to about 100 micrometers, and preferably between about 0.5 micrometers and about 25 micrometers.

Latent reactive reagents for providing a barrier of this invention optionally carry one or more pendent latent reactive (preferably photoreactive) groups covalently bonded to the polymer backbone. Alternatively, such photoreactive groups can be provided by the support surface itself, or by suitable linking reagents. Photoreactive groups are defined herein, and preferred groups are sufficiently stable to be stored under conditions in which they retain such properties. See, e.g., U.S. Patent No. 5,002,582. Latent reactive groups can be chosen that are responsive to various portions of the electromagnetic spectrum, with those responsive to ultraviolet and visible portions of the spectrum (referred to herein as "photoreactive") being particularly preferred.

Photoreactive groups respond to specific applied external stimuli to undergo active specie generation with resultant covalent bonding to an adjacent chemical structure, e.g., as provided by the same or a different molecule. Photoreactive groups are those groups of atoms in a molecule that retain their covalent bonds unchanged under conditions of storage but that, upon activation by an external energy source, form covalent bonds with other molecules.

The photoreactive groups generate active species such as free radicals and particularly nitrenes, carbenes, and excited states of ketones upon absorption of electromagnetic energy. Photoreactive groups may be chosen to be responsive to various portions of the electromagnetic spectrum, and photoreactive groups that are responsive to e.g., ultraviolet and visible portions of the spectrum are preferred and may be referred to herein occasionally as "photochemical group" or "photogroup".

Photoreactive aryl ketones are preferred, such as acetophenone, benzophenone, anthraquinone, anthrone, and anthrone-like heterocycles (i.e., heterocyclic analogs of anthrone such as those having N, O, or S in the 10- position), or their substituted (e.g., ring substituted) derivatives. The functional groups of such ketones are preferred since they are readily capable of undergoing the activation/inactivation/reactivation cycle described herein. Benzophenone is a particularly preferred photoreactive moiety, since it is capable of photochemical excitation with the initial formation of an excited singlet state that undergoes intersystem crossing to the triplet state. The excited triplet state can insert into carbon-hydrogen bonds by abstraction of a hydrogen atom (from a support surface, for example), thus creating a radical pair. Subsequent collapse of the radical pair leads to formation of a new carbon-carbon bond. If a reactive bond (e.g., carbon-hydrogen) is not available for bonding, the ultraviolet light-induced excitation of the benzophenone group is reversible and the molecule returns to ground state energy level upon removal of the energy source. Photoactivatible aryl ketones such as benzophenone and acetophenone are of particular importance inasmuch as these groups are subject to multiple reactivation in water and hence provide increased coating efficiency. Hence, photoreactive aryl ketones are particularly preferred.

The azides constitute a preferred class of photoreactive groups and include arylazides (C₆R₅N₃) such as phenyl azide and particularly 4-fluoro-3-nitrophenyl azide, acyl azides (-CO-N₃) such as benzoyl azide and p-methylbenzoyl azide, azido formates (-O-CO-N₃) such as ethyl azidoformate, phenyl azidoformate, sulfonyl azides (-SO₂-N₃) such as benzenesulfonyl azide, and phosphoryl azides (RO)₂PON₃ such as diphenyl phosphoryl azide and diethyl phosphoryl azide. Diazo compounds constitute another class of photoreactive groups and include diazoalkanes (-CHN₂) such as diazomethane and diphenyldiazomethane, diazoketones (-CO-CHN₂) such as diazoacetophenone and 1-trifluoromethyl-1-diazo-2-pentanone, diazoacetates (-O-CO-CHN₂) such as t-butyl diazoacetate and phenyl diazoacetate, and beta-keto-alpha-diazoacetates (-CO-CN₂-COO-) such as t-butyl alpha diazoacetoacetate. Other photoreactive groups include the diazirines (-CHN₂) such as 3-trifluoromethyl-3-phenyldiazirine, and ketenes (-CH=C=O) such as ketene and diphenylketene.

Upon activation of the photoreactive groups, the reagent molecules are covalently bound to each other and/or to the material surface by covalent bonds through residues of the photoreactive groups. Exemplary photoreactive groups, and their residues upon activation, are shown as follows.

| Photoreactive Functionality | Group | Residue |
|---|---|---|
| aryl azides | amine | R-NH-R' |
| acyl azides | amide | R-CO-NH-R' |
| azidoformates | carbamate | R-O-CO-NH-R' |
| sulfonyl azides | sulfonamide | R-SO₂-NH-R' |
| phosphoryl azides | phosphoramide | (RO)₂PO-NH-R' |
| diazoalkanes | new C-C bond | |
| diazoketones | new C-C bond and ketone | |
| diazoacetates | new C-C bond and ester | |
| beta-keto-alpha-diazoacetates | new C-C bond and beta-ketoester | |
| aliphatic azo | new C-C bond | |
| diazirines | new C-C bond | |
| ketenes | new C-C bond | |
| photoactivated ketones | new C-C bond and alcohol | |

One or more latent reactive groups can be attached to barrier-forming reagents in any suitable manner. Preferably the latent reactive groups are themselves covalently attached to the reagent, either directly or via linking groups. A coating composition of this invention can be prepared by any suitable means, e.g., by providing a barrier -forming molecule with one or more latent reactive groups, incorporated before or after its preparation. For instance, a complete barrier forming molecule can be derivatized with one or more latent reactive groups by covalently attaching the latent reactive group either at a reactive or functionalized end of a molecule, or at a reactive or functionalized pendant position. Barrier forming molecules frequently possess hydroxyl, or other reactive functionalities on either end of the molecule. Less frequently, these same functionalities branch off the main polymer backbone and can also be derivatized with latent reactive groups.

The invention will be further described with reference to the following nonlimiting Example. Unless otherwise indicated, all percentages are by weight.

### EXAMPLE

An experiment was performed to evaluate the use of an ethylene oxide/propylene oxide block copolymer and a photopolyvinylpyrrolidone copolymer as barriers of the present invention.

Stents were coated with a bioactive releasing composition and a barrier was provided in the manner described herein in order to determine its effectiveness. Prior to coating, all stents (LaserAge Technology Corporation, Waukegan, IL), 18mm length and 6 cell design, were cleaned for ten minutes in 3% Valtron SP2200 Alkaline Detergent (Valtech Corporation, Pughtown, PA) in an ultrasonic bath at 50°C. After cleaning the stents were rinsed with a three-stage deionized water cascade rinse for 5 minutes per stage. After rinsing, the stents were dried at 110°C for approximately one hour.

A polymeric coating solution was prepared for coating each stent. The solution was made from a mixture of 90 micrograms of pEVA (33 weight percent vinyl acetate, from Aldrich Chemical Company, Inc.) and 10 micrograms ofpoly(n-butyl methacrylate "pBMA") (337,000 average molecular weight, from Aldrich Chemical Company, Inc.) dissolved in tetrahydrofuran. All of the stents were coated with 500-800 micrograms of the polymeric coating solution using an IVEK sprayer composed of an IVEK Digispense 2000 System with a 0.04 inches (1.02 mm) orifice SonicAir Sprayhead (IVEK Corporation, North Springfield, Vermont) spraying at 4.5 psi (0.32 kg/cm²).

A commercially available ethylene oxide/propylene oxide block copolymer (Lutrol F127", BASF), 5mg/ml solution in ethanol was applied over the pEVA/pBMA polymeric coating on stent samples using the IVEK sprayer system at 4.5 psi (0.32 kg/cm²).

A photopolyvinylpyrrolidone copolymer ("PV05", SurModics, Inc., Eden Prairie, MN) 15mg/ml solution in DI water was applied over the pEVA/pBMA polymeric coating using the IVEK spraying at 10 psi (0.7 kg/cm²) followed by 30 minutes of drying. An Oriel UV light (Thermo Oriel Instruments, Stratford, CT) was positioned at a distance of 12 cm to cure the PV05 composition for approximately 20 minutes.

For mechanical testing, the stents were each crimped onto corresponding 4mm balloon catheters (Part No. 16901191, AngioDynamics, Inc., Enniscorthy, Ireland) using a radial-crimping tool (Machine Solutions, Inc., Flagstaff, AZ). A new balloon catheter was used for each group of stents. Prior to crimping, the balloon was compressed to the smallest size possible. After the stent was crimped onto the balloon, the assembly was placed in 37°C DI water for approximately 10 minutes. The balloon was inflated to 16 atm (16.5 kg/cm²), or 4 mm, then deflated and the stent was removed. After drying, the stent coating was examined for defects using a microscope at 50x. Delamination was defined as the coating pulling away from the surface of the stent.

**Table 1.**

| Stent Examples | Barrier Layer | Delamination | Comments |
|---|---|---|---|
| Comparative 1 | None | + | Delamination visible |
| Comparative 2 | None | + | Delamination visible |
| Comparative 3 | None | + | Delamination visible |
| 1 | Lutrol | - | |
| 2 | Lutrol | - | |
| 3 | Lutrol | - | |
| 4 | Lutrol | - | |
| 5 | PV05 | - | |
| 6 | PV05 | - | |
| 7 | PV05 | - | |

| | | | |
|---|---|---|---|
| Delamination was evaluated as (+) pulling, tearing, or delamination of the polymeric coating from the stent surface. | | | |

## Claims

1. A barrier adapted to be positioned between a first surface provided in the form of a polymeric, bioactive agent-containing coating upon an implantable medical device, and a second surface provided by another material positioned in apposition, and preferably moveable apposition, to the first surface, the barrier being selected from the group consisting of block copolymers and polymers bearing latent reactive groups, wherein the barrier prevents damage to and/or delamination of the polymeric, bioactive agent-containing coating.

2. A barrier according to claim 1 wherein the polymeric, bioactive agent-containing coating comprises a plurality of polymers.

3. A barrier according to claim 1 wherein the block copolymers are selected from ethylene oxide/propylene oxide block copolymers.

4. A barrier according to claim 1 wherein the polymers bearing latent reactive groups are selected from natural polymers selected from photoderivatized polysaccharides and polypeptides, and synthetic polymers selected from photoderivatized polyolefins, vinyl chloride polymers, fluorine-containing polymers, poly(vinyl acetates), poly(vinyl alcohols), poly(vinyl acetals), polyacrylates and polymethacrylates, styrene polymers and copolymers, vinyl thermoplastics, polyamides and polyimides, polyacetals, polycarbonates, thermoplastics containing p-phenylene groups, polyesters, polyurethanes, polyisocyanurates, and silicones.

5. A barrier according to claim 4 wherein the photoderivatized polymer is selected from photoderivatized polysaccharides, photoderivatized polyamides, and photoderivatized vinyl thermoplastics.

6. A barrier according to claim 5 wherein the photoderivatized polysaccharides are selected from photohyaluronic acid and photoheparin.

7. A barrier according to claim 5 wherein the photoderivatized polyamides comprise photoderivatized polyacrylamide copolymers, and the photoderivatized vinyl thermoplastics comprise photopolyvinylpyrrolidone.

8. A barrier according to claim 1 wherein the medical device is selected from the group consisting of vascular devices, orthopedic devices, dental devices, drug delivery devices, ophthalmic devices, urological devices, and synthetic prostheses, and the second surface is provided by another contacting portion of the same device or as a different material contained within or surrounding the device.

9. A barrier according to claim 8 wherein the medical device comprises a balloon-expandable stent, and the second surface is provided by an expandable balloon contained within the stent.

10. A barrier according to claim 1 wherein the bioactive agent within the polymeric coatings is present at a concentration of at least 20% based on the weight of the coated composition.

11. A barrier according to claim 10 wherein the bioactive agent within the polymeric coatings is present at a concentration of at least 30% based on the weight of the coated composition.

12. A barrier according to claim 11 wherein the bioactive agent within the polymeric coatings is present at a concentration of at least 40% based on the weight of the coated composition.

13. A barrier according to claim 1 wherein the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

14. A barrier according to claim 2 wherein the plurality of polymers comprises a first polymer selected from the group consisting of polyalkyl(meth)acrylate, polyaryl(meth)acrylates, polyaralkyl(meth)acrylates, and polyaryloxyalkyl(meth)acrylates, and a second polymer selected from the group consisting of poly(ethylene-co-vinyl acetate).

15. A barrier according to claim 14 wherein the polyalkyl(meth)acrylates comprise poly(n-butyl methacrylate) and the polyaryl(meth)acrylates are selected from poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate, the polyaralkyl(meth)acrylates are selected from polybenzylacrylate and methacrylate, poly-2-phenethylacrylate and methacrylate, poly-1-pyrenylmethylmethacrylate, and the polyaryloxyalkyl(meth)acrylates are selected from polyphenoxyethylacrylate and methacrylate, polyethyleneglycolphenylether acrylates and methacrylates with varying polyethyleneglycol molecular weights.

16. A barrier according to claim 1 wherein:
a) the polymeric, bioactive agent-containing coating comprises a plurality of polymers,
b) the block copolymers are selected from ethylene oxide/propylene oxide block copolymers,
c) the polymers bearing latent reactive groups are selected from photoderivatized polysaccharides and photoderivatized polyolefins, vinyl chloride polymers, fluorine-containing polymers, poly(vinyl acetates), poly(vinyl alcohols), poly(vinyl acetals), polyacrylates and polymethacrylates, styrene polymers and copolymers, vinyl thermoplastics, polyamides and polyimides, polyacetals, polycarbonates, thermoplastics containing p-phenylene groups, polyesters, polyurethanes, polyisocyanurates, and silicones,
d) the medical device comprises an implantable medical device,
e) the bioactive agent within the polymeric coatings is present at a concentration of at least 20% based on the weight of the coated composition, and
f) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

17. A barrier according to claim 16 wherein:
a) the polymeric, bioactive agent-containing coating comprises a plurality of polymers comprising a first polymer selected from the group consisting of polyalkyl(meth)acrylate, polyaryl(meth)acrylates, polyaralkyl(meth)acrylates, and polyaryloxyalkyl(meth)acrylates, and a second polymer selected from the group consisting of poly(ethylene-co-vinyl acetate),
b) the block copolymers are selected from ethylene oxide/propylene oxide block copolymers,
c) the polymers bearing latent reactive groups are selected from photoderivatized heparin, photoderivatized polyamides and photoderivatized vinyl thermoplastics,
d) the medical device is selected from the group consisting of vascular devices, orthopedic devices, dental devices, drug delivery devices, ophthalmic devices, urological devices, and synthetic prostheses,
e) the bioactive agent within the polymeric coatings is present at a concentration of at least 20% based on the weight of the coated composition, and
f) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

18. A barrier according to claim 1 wherein:
a) the polymeric, bioactive agent-containing coating comprises a plurality of polymers comprising a first polymer selected from the group consisting of polyalkyl(meth)acrylates, polyaryl(meth)acrylates, polyaralkyl(meth)acrylates, and polyaryloxyalkyl(meth)acrylates, and a second polymer selected from the group consisting of poly(ethylene-co-vinyl acetate),
b) the block copolymers are selected from ethylene oxide/propylene oxide block copolymers,
c) the polymers bearing latent reactive groups comprise a photoderivatized heparin, polyacrylamide, or polyvinylpyrrolidone,
d) the medical device comprises an implantable medical device selected from the group consisting of vascular devices, orthopedic devices, dental devices, drug delivery devices, ophthalmic devices, urological devices, and synthetic prostheses, and the second surface is provided by another contacting portion of the same device or as a different material contained within or surrounding the device,
e) the bioactive agent within the polymeric coatings is present at a concentration of at least 20% based on the weight of the coated composition, and
f) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

19. A barrier according to claim 1, wherein:
a) the block copolymers are selected from ethylene oxide/propylene oxide block copolymers,
b) the polymers bearing latent reactive groups are selected from photoderivatized polysaccharides, polyolefins, vinyl chloride polymers, fluorine-containing polymers, poly(vinyl acetates), poly(vinyl alcohols), poly(vinyl acetals), polyacrylates and polymethacrylates, styrene polymers and copolymers, vinyl thermoplastics, polyamides and polyimides, polyacetals, polycarbonates, thermoplastics containing p-phenylene groups, polyesters, polyurethanes, polyisocyanurates, and silicones,
c) the medical device comprises a balloon-expandable stent, and the second surface is provided by an expandable balloon contained within the stent,
d) the bioactive agent within the polymeric coatings is present at a concentration of at least 20% based on the weight of the coated composition,
e) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents, and
f) the polymeric, bioactive agent-containing coating comprises a plurality of polymers, comprising a first polymer selected from the group consisting of polyalkyl(meth)acrylates, polyaryl(meth)acrylates, polyaralkyl(meth)acrylates, and polyaryloxyalkyl(meth)acrylates, and a second polymer selected from the group consisting of poly(ethylene-co-vinyl acetate), wherein the polyalkyl(meth)acrylates comprise poly(n-butyl methacrylate) and the polyaryl(meth)acrylates are selected from poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate, the polyaralkyl(meth)acrylates are selected from polybenzylacrylate and methacrylate, poly-2-phenethylacrylate and methacrylate, poly-1-pyrenylmethylmethacrylate, and the polyaryloxyalkyl(meth)acrylates are selected from polyphenoxyethylacrylate and methacrylate, polyethyleneglycolphenylether acrylates and methacrylates with varying polyethyleneglycol molecular weights.

20. A combination comprising:
a) an implantable medical device having a first surface bearing a polymeric, bioactive agent-containing coating,
b) a second surface provided by another material positioned in apposition to the medical device, and
c) a barrier positioned between the first surface and the second surface, the barrier being selected from the group consisting of block copolymers and polymers bearing latent reactive groups, wherein the barrier prevents damage to and/or delamination of the polymeric, bioactive agent-containing coating.

21. A combination according to claim 20 wherein the barrier is provided in the form of a coating upon the polymeric coating, a coating upon the second surface, and/or a discrete layer positioned between the two.

22. A combination according to claim 20 wherein the barrier is itself comprised of one or more layers of the same or different materials, and positioned in any suitable combination upon the first and/or second surfaces, or separately provided between the two.

23. A combination according to claim 20 wherein the barrier is applied in the course of fabrication, storage, delivery or deployment, and/or residence of the device within the body.

24. A combination according to claim 20 wherein the polymeric, bioactive agent-containing coating is positioned upon the surface of an implantable medical device, the second surface is provided by the surface of an different material in apposition to the device, and the barrier comprises a barrier in the form of an anti-adherent coating adapted to facilitate the placement of the medical device surface and the different material in stable and separable apposition to each other.

25. A combination according to claim 24 wherein the medical device comprises a balloon-expandable stent, and the different material is in the form of an expandable balloon within the stent, and the barrier is selected from the group consisting of block copolymers and polymers bearing latent reactive groups.

26. A combination according to claim 20, wherein:
a) the polymeric, bioactive agent-containing coating comprises a plurality of polymers,
b) the block copolymers are selected from ethylene oxide/propylene oxide block copolymers,
c) the polymers bearing latent reactive groups are selected from photoderivatized polysaccharides and photoderivatized polyolefins, vinyl chloride polymers, fluorine-containing polymers, poly(vinyl acetates), poly(vinyl alcohols), poly(vinyl acetals), polyacrylates and polymethacrylates, styrene polymers and copolymers, vinyl thermoplastics, polyamides and polyimides, polyacetals, polycarbonates, thermoplastics containing p-phenylene groups, polyesters, polyurethanes, polyisocyanurates, and silicones,
d) the medical device comprises an implantable medical device,
e) the bioactive agent within the polymeric coatings is present at a concentration of at least 20% based on the weight of the coated composition, and
f) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

27. A composition according to claim 26 wherein:
a) the polymeric, bioactive agent-containing coating comprises a plurality of polymers comprising a first polymer selected from the group consisting of polyalkyl(meth)acrylates, polyaryl(meth)acrylates, polyaralkyl(meth)acrylates, and polyaryloxyalkyl(meth)acrylates, and a second polymer selected from the group consisting of poly(ethylene-co-vinyl acetate),
b) the block copolymers are selected from ethylene oxide/propylene oxide block copolymers,
c) the polymers bearing latent reactive groups are selected from photoderivatized heparin, photoderivatized polyamides and photoderivatized vinyl thermoplastics,
d) the medical device is selected from the group consisting of vascular devices, orthopedic devices, dental devices, drug delivery devices, ophthalmic devices, urological devices, and synthetic prostheses,
e) the bioactive agent within the polymeric coatings is present at a concentration of at least 20% based on the weight of the coated composition, and
f) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

28. A composition according to claim 20 wherein:
a) the polymeric, bioactive agent-containing coating comprises a plurality of polymers comprising a first polymer selected from the group consisting of polyalkyl(meth)acrylates, polyaryl(meth)acrylates, polyaralkyl(meth)acrylates, and polyaryloxyalkyl(meth)acrylates, and a second polymer selected from the group consisting of poly(ethylene-co-vinyl acetate),
b) the block copolymers are selected from ethylene oxide/propylene oxide block copolymers,
c) the polymers bearing latent reactive groups comprise a photoderivatized heparin, polyacrylamide, or polyvinylpyrrolidone,
d) the medical device comprises an implantable medical device selected from the group consisting of vascular devices, orthopedic devices, dental devices, drug delivery devices, ophthalmic devices, urological devices, and synthetic prostheses, and the second surface is provided by another contacting portion of the same device or as a different material contained within or surrounding the device,
e) the bioactive agent within the polymeric coatings is present at a concentration of at least 20% based on the weight of the coated composition, and
f) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents.

29. A combination according to claim 20, wherein:
a) the block copolymers are selected from ethylene oxide/propylene oxide block copolymers,
b) the polymers bearing latent reactive groups are selected from photoderivatized polysaccharides, polyolefins, vinyl chloride polymers, fluorine-containing polymers, poly(vinyl acetates), poly(vinyl alcohols), poly(vinyl acetals), polyacrylates and polymethacrylates, styrene polymers and copolymers, vinyl thermoplastics, polyamides and polyimides, polyacetals, polycarbonates, thermoplastics containing p-phenylene groups, polyesters, polyurethanes, polyisocyanurates, and silicones,
c) the medical device comprises a balloon-expandable stent, and the second surface is provided by an expandable balloon contained within the stent,
d) the bioactive agent within the polymeric coatings is present at a concentration of at least 20% based on the weight of the coated composition,
e) the bioactive agent is selected from the group consisting of thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, antisense nucleotides, anti metabolites, antiproliferatives (including antiangiogenesis agents), anticancer chemotherapeutic agents, steroidal or non-steroidal anti-inflammatory agents, immunosuppressive agents, growth hormone antagonists, growth factors, dopamine agonists, radiotherapeutic agents, peptides, proteins, enzymes, extracellular matrix components, ACE inhibitors, free radical scavengers, chelators, antioxidants, anti polymerases, antiviral agents, photodynamic therapy agents, and gene therapy agents, and
f) the polymeric, bioactive agent-containing coating comprises a plurality of polymers, comprising a first polymer selected from the group consisting of polyalkyl(meth)acrylates, polyaryl(meth)acrylates, polyaralkyl(meth)acrylates, and polyaryloxyalkyl(meth)acrylates, and a second polymer selected from the group consisting of poly(ethylene-co-vinyl acetate), wherein the polyalkyl(meth)acrylates comprise poly(n-butyl methacrylate) and the polyaryl(meth)acrylates are selected from poly-9-anthracenylmethacrylate, polychlorophenylacrylate, polymethacryloxy-2-hydroxybenzophenone, polymethacryloxybenzotriazole, polynaphthylacrylate, polynaphthylmethacrylate, poly-4-nitrophenylacrylate, polypentachloro(bromo, fluoro)acrylate and methacrylate, polyphenylacrylate and methacrylate, the polyaralkyl(meth)acrylates are selected from polybenzylacrylate and methacrylate, poly-2-phenethylacrylate and methacrylate, poly-1-pyrenylmethylmethacrylate, and the polyaryloxyalkyl(meth)acrylates are selected from polyphenoxyethylacrylate and methacrylate, polyethyleneglycolphenylether acrylates and methacrylates with varying polyethyleneglycol molecular weights.

30. Use of a barrier according to claim 1 for minimizing the damage caused to a first surface comprising polymeric, bioactive agent-containing composition upon a medical device surface, by a second surface provided by another material and positioned in apposition to the first surface.

## Patentansprüche

1. Sperrschicht, derart ausgestaltet, dass sie zwischen einer ersten Oberfläche, bereitgestellt in der Form einer polymeren, ein bioaktives Mittel enthaltenden Beschichtung auf einem implantierbaren medizinischen Gerät, und einer zweiten Oberfläche, bereitgestellt von einem anderen Material, welches in Apposition, und vorzugsweise in beweglicher Apposition, zu der ersten Oberfläche angeordnet ist, angeordnet werden kann, wobei die Sperrschicht ausgewählt ist aus der Gruppe, die aus Blockcopolymeren und Polymeren, die latent reaktive Gruppen tragen, besteht, wobei die Sperrschicht eine Beschädigung an und/oder ein Ablösen der polymeren, ein bioaktives Mittel enthaltenden Beschichtung verhindert.

2. Sperrschicht gemäß Anspruch 1, **dadurch gekennzeichnet, dass** die polymere, ein bioaktives Mittel enthaltende Beschichtung eine Vielzahl an Polymeren umfasst.

3. Sperrschicht gemäß Anspruch 1, **dadurch gekennzeichnet, dass** die Blockcopolymere aus Ethylenoxid/Propylenoxid-Biockcopolymeren ausgewählt sind.

4. Sperrschicht gemäß Anspruch1, **dadurch gekennzeichnet, dass** die Polymere, die latent reaktive Gruppen tragen, aus natürlichen Polymeren, ausgewählt aus photoderivatisierten Polysacchariden und Polypeptiden, und synthetischen Polymeren, ausgewählt aus photoderivatisierten Polyolefinen, Vinylchloridpolymeren, Fluor-haltigen Polymeren, Poly(vinylacetaten), Poly(vinylalkoholen), Poly(vinylacetalen), Polyacrylaten und Polymethacrylaten, Styrolpolymeren und -copolymeren, Vinylthermoplasten, Polyamiden und Polyimiden, Polyacetalen, Polycarbonate, Thermoplasten, enthaltend p-Phenylengruppen, Polyestern, Polyurethanen, Polyisocyanuraten und Siliconen ausgewählt sind.

5. Sperrschicht gemäß Anspruch 4, **dadurch gekennzeichnet, dass** das photoderivatisierte Polymer aus photoderivatisierten Polysacchariden, photoderivatisierten Polyamiden und photoderivatisierten Vinylthermoplasten ausgewählt ist.

6. Sperrschicht gemäß Anspruch 5, **dadurch gekennzeichnet, dass** die photoderivatisierten Polysaccharide aus Photohyaluronsäure und Photoheparin ausgewählt sind.

7. Sperrschicht gemäß Anspruch 5, **dadurch gekennzeichnet, dass** die photoderivatisierten Polyamide photoderivatisierte Polyacrylamidcopolymere und die photoderivatisierten Vinylthermoplasten Photopolyvinylpyrrolidon umfassen.

8. Sperrschicht gemäß Anspruch 1, **dadurch gekennzeichnet, dass** das medizinische Gerät aus der Gruppe bestehend aus vaskulären Geräten, orthopädischen Geräten, zahnmedizinischen Geräte, Geräten zur Bereitstellung von Arzneimitteln, ophthalmischen Geräten, urologischen Geräte und synthetischen Prothesen ausgewählt ist und die zweiten Oberfläche durch einen weiteren, kontaktierenden Bereich des selben Geräts oder als ein verschiedenes Material, welches in dem Gerät enthalten ist oder dieses umgibt, bereitgestellt wird.

9. Sperrschicht gemäß Anspruch 8, **dadurch gekennzeichnet, dass** das medizinische Gerät einen Ballon-dehnbaren Stent umfasst und die zweiten Oberfläche durch einen dehnbaren Ballon, der in dem Stent enthalten ist, bereit gestellt wird.

10. Sperrschicht gemäß Anspruch 1, **dadurch gekennzeichnet, dass** das bioaktive Mittel in den polymeren Beschichtungen in einer Konzentration von mindestens 20 Gew.-%, bezogen auf das Gewicht der beschichteten Zusammensetzung, vorhanden ist.

11. Sperrschicht gemäß Anspruch 10, **dadurch gekennzeichnet, dass** das bioaktive Mittel in den polymeren Beschichtungen in einer Konzentration von mindestens 30 Gew.-%, bezogen auf das Gewicht der beschichteten Zusammensetzung, vorhanden ist.

12. Sperrschicht gemäß Anspruch 11, **dadurch gekennzeichnet, dass** das bioaktive Mittel in den polymeren Beschichtungen in einer Konzentration von mindestens 40 Gew.-%, bezogen auf das Gewicht der beschichteten Zusammensetzung, vorhanden ist.

13. Sperrschicht gemäß Anspruch 1, **dadurch gekennzeichnet, dass** das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmittein, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa (einschließlich Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist.

14. Sperrschicht gemäß Anspruch 2, **dadurch gekennzeichnet, dass** die Vielzahl an Polymeren ein erstes Polymer, welches aus der Gruppe ausgewählt ist, die aus Polyalkyl(meth)acrylat, Polyaryl(meth)acrylaten, Polyaralkyl(meth)acrylaten und Polyaryloxyalkyl(meth)acrylaten besteht, umfasst und ein zweites Polymer, weiches aus der Gruppe ausgewählt ist, die aus Poly(ethylen-co-vinylacetat) besteht, umfasst.

15. Sperrschicht gemäß Anspruch 14, **dadurch gekennzeichnet, dass** die Polyalkyl(meth)acrylate Poly(n-butyl methacrylat) umfassen und die Polyaryl(meth)acrylate aus Poly-9-anthracenylmethacrylat, Polychlorphenylacrylat, Polymethacryloxy-2-hydroxybenzophenon, Polymethacryloxybenzotriazol, Polynaphthylacrylat, Polynaphthylmethacrylat, Poly-4-nitrophenylacrylat, Polypentachlor(brom,fluor)acrylat und -methacrylat, Polyphenylacrylat und - methacrylat ausgewählt sind, die Polyaralkyl(meth)acrylate ausgewahlt sind aus der Gruppe, die aus Polybenzylacrylat und -methacrylat, Poly-2-phenethylacrylat und -methacrylat, Poly-1-pyrenylmethylmethacrylat besteht, und die Polyaryloxyalkyl(meth)acrylate ausgewählt sind aus der Gruppe, die aus Polyphenoxyethylacrylat und -methacrylat, Polyethylenglykolphenyletheracrylaten und -methacrylaten mit variierenden molekularen Gewichten an Polyethylenglycol besteht.

16. Sperrschicht gemäß Anspruch 1, **dadurch gekennzeichnet, dass**
a) die polymere, ein bioaktives Mittel enthaltenden Beschichtung eine Vielzahl an Polymeren enthält,
b) die Blockcopolymere aus Ethylenoxid/Propylenoxid-Blockcopolymeren ausgewählt sind,
c) die Polymere, die latent reaktive Gruppen tragen, aus photoderivatisierten Polysacchariden, photoderivatisierten Polyolefinen, Vinylchloridpolymeren, Fluor-haltigen Plymeren, Poly(vinylacetaten), Poly(vinyialkoholen), Poly(vinylacetalen), Polyacrylaten und Polymethacrylaten, Styrolpolymeren und -copolymeren, Vinylthermoplasten, Polyamide und Polyimide, Polyacetalen, Polycarbonaten, Thermoplasten, enthaltend p-Phenylengruppen, Polyestern, Polyurethanen, Polyisocyanuraten und Siliconen ausgewählt sind,
d) das medizinische Gerät ein implantierbares medizinische Gerät umfasst,
e) das bioaktive Mittel in den polymeren Beschichtungen in einer Konzentration von mindestens 20 Gew.-%, bezogen auf das Gewicht der beschichteten Zusammensetzung, vorhanden ist und
f) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiprotiferativa (einschließlich Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist.

17. Sperrschicht gemäß Anspruch 16, **dadurch gekennzeichnet, dass**
a) die polymere, ein bioaktives Mittel enthaltende Beschichtung eine Vielzahl an Polymeren umfasst, die ein erstes Polymer, welches aus der Gruppe ausgewählt ist, die aus Polyalkyl(meth)acrylat, Polyaryl(meth)acrylaten, Polyaralkyl(meth)acrylate und Polyaryloxyalkyl(meth)acrylaten besteht, umfasst und ein zweites Polymer, welches aus der Gruppe ausgewählt ist, die aus Poly(ethylen-co-vinylacetat) besteht, umfasst,
b) die Blockcopolymere aus Ethylenoxid/Propylenoxid-Blockcopolymeren ausgewählt sind,
c) die Polymere, die latent reaktive Gruppen tragen, ausgewählt sind aus photoderivatisierten Heparin, photoderivatisierten Polyamiden und photoderivatisierten Vinylthermoplasten, d) das medizinische Gerät aus der Gruppe bestehend aus vaskulären Geräten, orthopädischen Geräten, zahnmedizinischen Geräten, Geräten zur Bereitstellung von Arzneimitteln, ophthalmischen Geräten, urologische Geräten und synthetischen Prothesen ausgewählt ist,
e) das bioaktive Mittel in den polymeren Beschichtungen in einer Konzentration von mindestens 20 Gew.-%, bezogen auf das Gewicht der beschichteten Zusammensetzung, vorhanden ist und
f) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa (einschließlich Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnem, Antioxidationsmitteln, Antipolymerasen, antiviralen Mitteln, Mitteln für photodynamische Therapie und Mitteln für Gentherapie ausgewählt ist.

18. Sperrschicht gemäß Anspruch 1, **dadurch gekennzeichnet, dass**
a) die polymere, ein bioaktives Mittel enthaltenden Beschichtung eine Vielzahl an Polymeren umfasst, die ein erstes Polymer, welches aus der Gruppe ausgewählt ist, die aus Polyalkyl(meth)acrylat, Polyaryl(meth)acrylaten, Polyaralkyl(meth)acrylate und Polyaryloxyalkyl(meth)acrylaten besteht, umfasst und ein zweites Polymer, welches aus der Gruppe ausgewählt ist, die aus Poly(ethylen-co-vinylacetat) besteht, umfasst,
b) die Blockcopolymere aus Ethylenoxid/Propylenoxid-Blockcopolymeren ausgewählt sind,
c) die Polymere, die latent reaktive Gruppen tragen, ausgewählt sind aus photoderivatisierten Heparin, Polyacrylamid oder Polyvinylpyrrolidon,
d) das medizinische Gerät ein implantierbares medizinische Gerät, aus der Gruppe bestehend aus vaskulären Geräten, orthopädischen Geräten, zahnmedizinischen Geräten, Geräten zur Bereitstellung von Arzneimitteln, ophthalmischen Geräten, urologischen Geräten, und synthetischen Prothesen ausgewählt ist, umfasst und die zweiten Oberfläche durch einen weiteren, kontaktierenden Bereich des selben Geräts oder als ein verschiedenes Material, welches in dem Gerät enthalten ist oder dieses umgibt, bereitgestellt wird,
e) das bioaktive Mittel in den polymeren Beschichtungen in einer Konzentration von mindestens 20 Gew.-%, bezogen auf das Gewicht der beschichteten Zusammensetzung, vorhanden ist,
f) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa (einschließlich. Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist.

19. Sperrschicht gemäß Anspruch 1, **dadurch gekennzeichnet, dass**: a) die Blockcopolymere aus Ethylenoxid/Propylenoxid-Blockcopolymeren ausgewählt sind, b) die Polymere, die latent reaktive Gruppen tragen, aus photoderivatisierten Polysacchariden Polyolefinen, Vinylchloridpolymeren, Fluor-haltigen Plymeren, Poly(vinylacetaten), Poly(vinylalkoholen), Poly(vinylacetalen), Polyacrylaten und Polymethacrylaten, Styrolpolymeren und -copolymeren, Vinylthermoplasten, Polyamide und Polyimide, Polyacetale, Polycarbonate, Thermoplasten, enthaltend p-Phenylengruppen, Polyestern, Polyurethanen, Polyisocyanuraten und Siliconen ausgewählt sind, c) das medizinische Gerät einen Ballon-dehnbaren Stent umfasst und die zweiten Oberfläche durch einen dehnbaren Ballon, der in dem Stent enthalten ist, bereit gestellt wird, d) das bioaktive Mittel in den polymeren Beschichtungen in einer Konzentration von mindestens 20 Gew.-%, bezogen auf das Gewicht der beschichteten Zusammensetzung, vorhanden ist, e) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretoriscnen Mitteln, Actin-Inhibitoren, Remodeling-Inhibitoren, Antisense-Nukleotiden, Antimetaboliten, Antiproliferativa (einschließlich Antiangiogenese-Mittein), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnem, Antioxidationsmitteln, Antipolymerasen, antiviralen Mitteln, Mitteln für photodynamische Therapie und Mitteln für Gentherapie ausgewählt ist und f) die polymere, ein bioaktives Mittel enthaltenden Beschichtung eine Vielzahl an Polymeren ein erstes Polymer, welches aus der Gruppe ausgewählt ist, die aus Polyalkyl(meth)acrylat, Polyaryl(meth)acrylaten, Polyaralkyl(meth)acrylate und Polyaryloxyalkyl(meth)acrylaten besteht, umfasst und ein zweites Polymer, welches aus der Gruppe ausgewählt ist, die aus Poly(ethylen-co-vinylacetat) besteht, umfasst, wobei die Polyalkyl(meth)acrylate Poly(n-butyl methacrylat) umfassen und die Polyaryl(meth)acrylate aus Poly-9-anthracenylmethacrylat, Polychlorphenylacrylat, Polymethacryloxy-2-hydroxybenzophenon, Polymethacryloxybenzotriazol, Polynaphthylacrylat, Polynaphthylmethacrylat, Poly-4-nitrophenylacrylat, Polypentachlor(brom,fluor)acrylat und -methacrylat, Polyphenylacrylat und - methacrylat ausgewählt sind; die Polyaralkyl(meth)acrylate ausgewählt sind aus der Gruppe, die aus Polybenzylacrylat und -methacrylat, Poly-2-phenethylacrylat und -methacrylat, Poly-1-pyrenylmethylmethacrylat besteht, und die Polyaryloxyalkyl(meth)acrylate ausgewählt sind aus der Gruppe, die aus Polyphenoxyethylacrylat und -methacrylat, Polyethylenglykolphenyletheracrylaten und -methacrylaten mit variierenden molekularen Gewichten an Polyethylenglycol besteht.

20. Kombination, enthaltend
a) ein implantierbares medizinisches Gerät mit einer ersten Oberfläche, die eine polymere, ein bioaktives Mittel enthaltende Beschichtung trägt,
b) eine zweite Oberfläche, die von einem anderen Material, welches in Apposition zu dem medizinischen Gerät angeordnet ist, bereitgestellt wird und
c) eine Sperrschicht, die zwischen der ersten Oberfläche und der zweiten Oberfläche angeordnet ist, wobei die Sperrschicht ausgewählt ist aus der Gruppe, die aus Blockcopolymeren und Polymeren, die latent reaktive Gruppen tragen, besteht, wobei die Sperrschicht eine Beschädigung an und/oder ein Ablösen der polymeren, ein bioaktives Mittel enthaltenden Beschichtung verhindert.

21. Kombination gemäß Anspruch 20, **dadurch gekennzeichnet, dass** die Sperrschicht in der Form einer Beschichtung auf der polymeren Beschichtung, einer Beschichtung auf der zweiten Oberfläche und/oder einer diskreten Schicht, die zwischen den beiden angeordnet ist, bereitgestellt wird.

22. Kombination gemäß Anspruch 20, **dadurch gekennzeichnet, dass** die Sperrschicht selber eine oder mehrere Schichten aus demselben Material oder aus unterschiedlichen Materialien umfasst und in jeglicher geeigneten Kombination auf der ersten und/oder zweiten Oberflächen angeordnet ist oder separat, zwischen den beiden bereitgestellt wird.

23. Kombination gemäß Anspruch 20, **dadurch gekennzeichnet, dass** die Sperrschicht im Zuge der Herstellung, der Lagerung, der Auslieferung oder des Einsatzes und/oder des Verbleibs des Geräts im Körper aufgebracht wird.

24. Kombination gemäß Anspruch 20, **dadurch gekennzeichnet, dass** die polymeren, ein bioaktives Mittel enthaltenden Beschichtung auf der Oberfläche eines implantierbaren, medizinischen Gerätes angeordnet ist, die zweite Oberfläche durch die Oberfläche eines unterschiedlichen Materials in Apposition zu dem Gerät bereitgestellt wird und die Sperrschicht umfasst eine Sperrschicht in Form einer nicht-anhaftenden Beschichtung, die derart ausgestaltet ist, dass sie die Platzierung der Oberfläche des medizinischen Geräts und dem verschiedenen Material in stabiler und trennbarer Apposition zu einander erleichtert.

25. Kombination gemäß Anspruch 24, **dadurch gekennzeichnet, dass** das medizinische Gerät einen Ballon-dehnbaren Stent umfasst und das unterschiedliche Material in der Form eines dehnbaren Ballons, der in dem Stent enthalten ist, vorliegt und die Sperrschicht ausgewählt ist aus der Gruppe, die aus Blockcopolymeren und Polymeren, die latent reaktive Gruppen tragen, besteht.

26. Kombination gemäß Anspruch 20, **dadurch gekennzeichnet, dass**
a) die polymere, ein bioaktives Mittel enthaltenden Beschichtung eine Vielzahl an Polymeren umfasst,
b) die Blockcopolymere aus Ethylenoxid/Propylenoxid-Blockcopolymeren ausgewählt sind,
c) die Polymere, die latent reaktive Gruppen tragen, aus photoderivatisierten Polysacchariden und photoderivatisierten Polyolefinen, Vinylchloridpolymeren, Fluor-haltigen Plymeren, Poly(vinylacetaten), Poly(vinylalkoholen), Poly(vinylacetalen), Polyacrylaten und Polymethacrylaten, Styrolpolymeren und -copolymeren, Vinylthermoplasten, Polyamide und Polyimide, Polyacetalen, Polycarbonaten, Thermoplasten, enthaltend p-Phenylengruppen, Polyestern, Polyurethanen, Polyisocyanuraten und Siliconen ausgewählt sind,
d) das medizinische Gerät ein implantierbares medizinisches Gerät umfasst,
e) das bioaktive Mittel in den polymeren Beschichtungen in einer Konzentration von mindestens 20 Gew.-%, bezogen auf das Gewicht der beschichteten Zusammensetzung, vorhanden ist und
f) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa (einschließlich Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist.

27. Zusammensetzung gemäß Anspruch 26, **dadurch gekennzeichnet, dass**:
a) die polymere, ein bioaktives Mittel enthaltende Beschichtung eine Vielzahl an Polymeren umfasst, die ein erstes Polymer, welches aus der Gruppe ausgewählt ist, die aus Polyalkyl(meth)acrylat, Polyaryl(meth)acrylaten, Polyaralkyl(meth)acrylate und Polyaryloxyalkyl(meth)acrylaten besteht, umfasst und ein zweites Polymer, welches aus der Gruppe ausgewählt ist, die aus Poly(ethylen-co-vinylacetat) besteht, umfasst,
b) die Blockcopolymere aus Ethylenoxid/Propylenoxid-Blockcopolymeren ausgewählt sind,
c) die Polymere, die latent reaktive Gruppen tragen, sind ausgewählt aus photoderivatisierten Heparin, photoderivatisierten Polyamiden und photoderivatisierten Vinylthermoplasten,
d) das medizinische Gerät aus der Gruppe bestehend aus vaskulären Geräten, orthopädischen Geräten, zahnmedizinischen Geräten, Geräten zur Bereitstellung von Arzneimitteln, ophthalmischen Geräten, urologischen Geräten und synthetischen Prothesen ausgewählt ist,
e) das bioaktive Mittel in den polymeren Beschichtungen in einer Konzentration von mindestens 20 Gew.-%, bezogen auf das Gewicht der beschichteten Zusammensetzung, vorhanden ist und
f) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa (einschließlich Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Mairixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist.

28. Zusammensetzung gemäß Anspruch 20, **dadurch gekennzeichnet, dass**
a) die polymere, ein bioaktives Mittel enthaltenden Beschichtung eine Vielzahl an Polymeren umfasst, die ein erstes Polymer, welches aus der Gruppe ausgewählt ist, die aus Polyalkyl(meth)acrylat, Polyaryl(meth)acrylaten, Polyaralkyl(meth)acrylate und Polyaryloxyalkyl(meth)acrylaten besteht, umfasst und ein zweites Polymer, welches aus der Gruppe ausgewählt ist, die aus Poly(ethylen-co-vinylacetat) besteht, umfasst,
b) die Blockcopolymere aus Ethylenoxid/Propylenoxid-Blockcopolymeren ausgewählt sind, c) die Polymere, die latent reaktive Gruppen tragen, ausgewählt sind aus photoderivatisiertem Heparin, Polyacrylamid oder Polyvinylpyrrolidon,
d) das medizinische Gerät ein implantierbares medizinische Gerät umfasst, welches aus der Gruppe bestehend aus vaskulären Geräten, orthopädischen Geräten, zahnmedizinischen Geräten, Geräten zur Bereitstellung von Arzneimitteln, ophthalmischen Geräten, urologischen Geräten und synthetischen Prothesen ausgewählt ist und die zweiten Oberfläche durch einen weiteren, kontaktierenden Bereich des selben Geräts oder als ein verschiedenes Material, welches in dem Gerät enthalten ist oder dieses umgibt, bereitgestellt wird,
e) das bioaktive Mittel in den polymeren Beschichtungen in einer Konzentration von mindestens 20 Gew.-%, bezogen auf das Gewicht der beschichteten Zusammensetzung, vorhanden ist und
f) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actinlnhibitoren, Remodeling-Inhibitoren, Antisense-Nukleotiden, Antimetaboliten, Antiproliferativa (einschließlich Antlanglogenese-Mitteln), chemotherapeutischen Antikrebsmitteln, entzündungshemmenden Steroiden oder nicht-steroiden entzündungshemmenden Mitteln, lmmunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist.

29. Kombination gemäß Anspruch 20, **dadurch gekennzeichnet, dass**
a) die Blockcopolymere aus Ethylenoxid/Propylenoxid-Blockcopolymeren ausgewählt sind,
b) die Polymere, die latent reaktive Gruppen tragen, aus photoderivatisierten Polysacchariden und photoderivatisierten Polyolefinen, Vinylchloridpolymeren, Fluor-haltigen Plymeren, Poly(vinylacetaten), Poly(vinylalkoholen), Poly(vinylacetalen), Polyacrylaten und Polymethacrylaten, Styrolpolymeren und -copolymeren, Vinylthermoplasten, Polyamide und Polyimide, Polyacetalen, Polycarbonaten, Thermoplasten, enthaltend p-Phenylengruppen, Polyestern, Polyurethanen, Polyisocyanuraten und Siliconen ausgewählt sind,
c) das medizinische Gerät einen Ballon-dehnbaren Stent umfasst und die zweiten Oberfläche durch einen dehnbaren Ballon, der in dem Stent enthalten ist, bereit gestellt wird,
d) das bioaktive Mittel in den polymeren Beschichtungen in einer Konzentration von mindestens 20 Gew.-%, bezogen auf das Gewicht der beschichteten Zusammensetzung, vorhanden ist,
e) das bioaktive Mittel aus der Gruppe, bestehend aus Thrombin-Inhibitoren, Antithrombosemitteln, thrombolytischen Mitteln, fibrinolytischen Mitteln, Vasospasmus-Inhibitoren, Calciumkanalblockern, Vasodilatatoren, Antihypertonika, antimikrobiellen Mitteln, Antibiotika, Inhibitoren von Oberflächen-Glykoprotein-Rezeptoren, Antithrombozytenmitteln, Antimitotika, Mikrotubuli-Inhibitoren, antisekretorischen Mitteln, Actin-Inhibitoren, Remodeling-Inhibitoren, Antisense-Nukleotiden, Antimetaboliten, Antiproliferativa (einschließlich Antiangiogenese-Mitteln), chemotherapeutischen Antikrebsmitteln, entzündungshemmenden Steroiden oder nicht-steroiden entzündungshemmenden Mitteln, Immunsuppressiva, Wachstumshormon-Antagonisten, Wachstumsfaktoren, Dopamin-Agonisten, Radiotherapeutika, Peptiden, Proteinen, Enzymen, extrazellulären Matrixkomponenten, Radikalfängern, Chelatbildnern, Antioxidationsmitteln, Antipolymerasen, antiviralen Mitteln, Mitteln für photodynamische Therapie und Mitteln für Gentherapie ausgewählt ist und
f) das polymeren, ein bioaktives Mittel enthaltenden Beschichtung Vielzahl an Polymeren ein erstes Polymer, welches aus der Gruppe ausgewählt ist, die aus Polyalkyl(meth)acrylat, Polyaryl(meth)acrylaten, Polyaralkyl(meth)acrylate und Polyaryloxyalkyl(meth)acrylaten besteht, umfasst und ein zweites Polymer, welches aus der Gruppe ausgewählt ist, die aus Poly(ethylen-co-vinylacetat) besteht, umfasst, wobei die Polyalkyl(meth)acrylate Poly(n-butylmethacrylat) umfassen und die Polyaryl(meth)acrylate aus Poly-9-anthracenylmethacrylat, Polychlorphenylacrylat, Polymethacryloxy-2-hydroxybenzophenon, Polymethacryloxybenzotriazol, Polynaphthylacrylat, Polynaphthylmethacrylat, Poly-4-nitrophenylacrylat, Polypentachlor(brom,fluor)acrylat und -methacrylat, Polyphenylacrylat und -methacrylat ausgewählt sind; die Polyaralkyl(meth)acrylate ausgewählt sind aus der Gruppe, die aus Polybenzylacrylat und -methacrylat, Poly-2-phenethylacrylat und -methacrylat, Poly-1-pyrenylmethylmethacrylat besteht, und die Polyaryloxyalkyl(meth)acrylate ausgewählt sind aus der Gruppe, die aus Polyphenoxyethylacrylat und -methacrylat, Polyethylenglykolphenyletheracrylaten und -methacrylaten mit variierenden molekularen Gewichten an Polyethylenglycol besteht.

30. Verwendung einer Sperrschicht gemäß Anspruch 1 zur Minimierung der Beschädigung einer ersten Oberfläche, die eine polymere, ein bioaktives Mittel enthaltende Beschichtung auf der Oberfläche eines medizinischen Geräts umfasst, durch eine zweite Oberfläche, die durch ein anderes Material bereitgestellt wird und in Apposition zu der ersten Oberfläche angeordnet ist.

## Revendications

1. Barrière adaptée destinée à être positionnée entre une première surface fournie sous la forme d'un revêtement polymère contenant un agent bioactif sur un dispositif médical implantable, et une seconde surface fournie par un autre matériau positionnée en apposition, et de préférence en apposition mobile, par rapport à la première surface, la barrière étant choisie dans le groupe se composant de copolymères blocs et de polymères portant des groupes réactifs latents, dans laquelle la barrière empêche que se produise(nt) des dommages et/ou un délaminage du revêtement polymère contenant un agent bioactif.

2. Barrière selon la revendication 1 dans laquelle le revêtement polymère contenant un agent bioactif comprend une pluralité de polymères.

3. Barrière selon la revendication 1 dans laquelle les copolymères blocs sont choisis parmi les copolymères blocs d'oxyde d'éthylène et d'oxyde de propylène.

4. Barrière selon la revendication 1 dans laquelle les polymères portant des groupes réactifs latents sont choisis parmi les polysaccharides et les polypeptides photodérivés, et les polymères synthétiques sont choisis parmi les polyoléfines photodérivées, les polymères de chlorure de vinyle photodérivés, les polymères contenant du fluor photodérivés, les acétates de polyvinyle photodérivés, les alcools polyvinyliques photodérivés, les polyacétals de vinyle photodérivés, les polyacrylates et les polyméthacrylates photodérivés, les polymères et les copolymères de styrène et les thermoplastiques vinyle photodérivés, les polyamides et les polyimides photodérivés, les polyacétals photodérivés, les polycarbonates photodérivés, les thermoplastiques contenant des groupes p-phénylène photodérivés, les polyesters photodérivés, les polyuréthanes photodérivés, les polyisocyanurates photodérivés, et les silicones photodérivées.

5. Barrière selon la revendication 4 dans laquelle le polymère photodérivé est choisi parmi les polysaccharides photodérivés, les polyamides photodérivés, et les thermoplastiques vinyle photodérivés.

6. Barrière selon la revendication 5 dans laquelle les polysaccharides photodérivés sont choisis parmi l'acide photohyaluronique et la photohéparine.

7. Barrière selon la revendication 5 dans laquelle les polyamides photodérivés comprennent des copolymères de polyacrylamide photodérivé, et les thermoplastiques vinyle photodérivés comprennent de la photopolyvinylpyrrolidone.

8. Barrière selon la revendication 1 dans laquelle le dispositif médical est choisi dans le groupe constitué par des dispositifs vasculaires, des dispositifs orthopédiques, des dispositifs dentaires, des dispositifs d'administration de produits médicamenteux, des dispositifs ophtalmiques, des dispositifs urologiques, et des prothèses synthétiques, et la seconde surface est fournie par une autre partie de contact du même dispositif ou en tant que matériau différent contenu à l'intérieur ou autour du dispositif.

9. Barrière selon la revendication 8 dans laquelle le dispositif médical comprend un stent à ballonnet dilatable, et la seconde surface est munie d'un ballonnet dilatable contenu à l'intérieur du stent.

10. Barrière selon la revendication 1 dans laquelle l'agent bioactif à l'intérieur des revêtements polymères est présent à une concentration d'au moins 20 % par rapport au poids de la composition revêtue.

11. Barrière selon la revendication 10 dans laquelle l'agent bioactif à l'intérieur des revêtements polymères est présent à une concentration d'au moins 30 % par rapport au poids de la composition revêtue.

12. Barrière selon la revendication 11 dans laquelle l'agent bioactif à l'intérieur des revêtements polymères est présent à une concentration d'au moins 40 % par rapport au poids de la composition revêtue.

13. Barrière selon la revendication 1 dans laquelle l'agent bioactif est choisi dans le groupe constitué par des inhibiteurs des thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, d'antimitotiques, des inhibiteurs des microtubules, des agents anti-secréteurs, des inhibiteurs d'actine, d'inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, des antagonistes de l'hormone de croissance, des facteurs de croissance, des agonistes de la dopamine, des agents radiothérapiques, des peptides, des protéines, des enzymes, des composants de la matrice extracellulaire, des inhibiteurs de l'ECA, des désactivateurs des radicaux libres, des chélateurs, des antioxydants, des antipolymérases, des agents antiviraux, des agents de thérapie photodynamique, et des agents de thérapie génique.

14. Barrière selon la revendication 2 dans laquelle la pluralité de polymères comprend un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe se composant du poly(éthylène-co-vinyle acétate).

15. Barrière selon la revendication 14 dans laquelle les polyalkyl(méth)acrylates comprennent le poly(n-butyl méthacrylate) et les polyaryl(méth)acrylates sont choisis parmi le poly-9-anthracénylméthacrylate, le polychlorophénylacrylate, le polyméthacryloxy-2-hydroxybenzophénone, le polyméthacryloxybenzotriazole, le polynaphtylacrylate, le polynaphtylméthacrylate, le poly-4-nitrophénylacrylate, les polypentachloro(bromo,fluoro)acrylate et méthacrylate, les polyphénylacrylate et méthacrylate, les polyaralkyl(méth)acrylates sont choisis parmi les polybenzylacrylate et méthacrylate, les poly-2-phénéthylacrylate et méthacrylate, le poly-1-pyrénylméthylméthacrylate, et les polyaryloxyalkyl(méth)acrylates sont choisis parmi les polyphénoxyéthylacrylate et méthacrylate, les polyéthyléne glycol phényl éther acrylates et méthacrylates ayant différentes masses moléculaires de polyéthylène glycol.

16. Barrière selon la revendication 1 dans laquelle :
a) le revêtement polymère contenant un agent bioactif comprend une pluralité de polymères,
b) les copolymères blocs sont choisis parmi les copolymères blocs d'oxyde d'éthylène et d'oxyde de propylène,
c) les polymères portant des groupes réactifs latents sont choisis parmi les polysaccharides photodérivés et les polyoléfines photodérivées, les polymères de chlorure de vinyle photodérivés, les polymères contenant du fluor photodérivés, les polyacétates de vinyle photodérivés, les alcools polyvinyliques photodérivés, les polyacétals de vinyle photodérivés, les polyacrylates et les polyméthacrylates photodérivés, les polymères et les copolymères de styrène photodérivés, les thermoplastiques vinyle photodérivés, les polyamides et les polyimides photodérivés, les polyacétals photodérivés, les polycarbonates photodérivés, les thermoplastiques contenant des groupes p-phénylene photodérivés, les polyesters photodérivés, les polyuréthanes photodérivés, les polyisocyanurates photodérivés, et les silicones photodérivées,
d) le dispositif médical comprend un dispositif médical implantable,
e) l'agent bioactif à l'intérieur des revêtements polymères est présent à une concentration d'au moins 20 % par rapport au poids de la composition revêtue, et
f) l'agent bioactif est choisi dans le groupe constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, des antagonistes de l'hormone de croissance, des facteurs de croissance, des agonistes de la dopamine, des agents radiothérapiques, des peptides, des protéines, des enzymes, des composants de la matrice extracellulaire, des inhibiteurs de l'ECA, des désactivateurs des radicaux libres, des chélateurs, des antioxydants, des antipolymérases, des agents antiviraux, des agents de thérapie photodynamique, et des agents de thérapie génique.

17. Barrière selon la revendication 16 dans laquelle :
a) le revêtement polymère contenant un agent bioactif comprend une pluralité de polymères comprenant un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe constitué par du poly(éthylène-co-vinyle acétate),
b) les copolymères blocs sont choisis parmi les copolymères blocs d'oxyde d'éthylène et d'oxyde de propylène,
c) les polymères portant des groupes réactifs latents sont choisis parmi l'héparine photodérivée, les polyamides photodérivés et les thermoplastiques vinyle photodérivés,
d) le dispositif médical est choisi dans le groupe est constitué par des dispositifs vasculaires, des dispositifs orthopédiques, des dispositifs dentaires, des dispositifs d'administration de produits médicamenteux, des dispositifs ophtalmiques, des dispositifs urologiques, et des prothèses synthétiques,
e) l'agent bioactif à l'intérieur des revêtements polymères est présent à une concentration d'au moins 20 % par rapport au poids de la composition revêtue, et
f) l'agent bioactif est choisi dans le groupe constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, d'antagonistes de l'hormone de croissance, des facteurs de croissance, des agonistes de la dopamine, des agents radiothérapiques, des peptides, des protéines, des enzymes, des composants de la matrice extracellulaire, des inhibiteurs de l'ECA, des désactivateurs des radicaux libres, des chélateurs, des antioxydants, des antipolymérases, des agents antiviraux, des agents de thérapie photodynamique, et des agents de thérapie génique.

18. Barrière selon la revendication 1 dans laquelle :
a) le revêtement polymère contenant un agent bioactif comprend une pluralité de polymères comprenant un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe se composant du poly(éthylène-co-vinyle acétate),
b) les copolymères blocs sont choisis parmi les copolymères blocs d'oxyde d'éthylène et d'oxyde de propylène,
c) les polymères portant des groupes réactifs latents comprennent une héparine phtodérivée, un polyacrylamide photodérivé, ou une polyvinylpyrrolidone photodérivée,
d) le dispositif médical comprend un dispositif médical implantable choisi dans le groupe constitué par des dispositifs vasculaires, des dispositifs orthopédiques, des dispositifs dentaires, des dispositifs d'administration de produits médicamenteux, des dispositifs ophtalmiques, des dispositifs urologiques, et des prothèses synthétiques, et la seconde surface est fournie par une autre partie de contact du même dispositif ou en tant que matériau différent contenu à l'intérieur ou autour du dispositif.
e) l'agent bioactif à l'intérieur des revêtements polymères est présent à une concentration d'au moins 20 % par rapport au poids de la composition revêtue, et
f) l'agent bioactif est choisi dans le groupe constitué par desinhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, des antagonistes de l'hormone de croissance, des facteurs de croissance, des agonistes de la dopamine, des agents radiothérapiques, des peptides, des protéines, des enzymes, de composants de la matrice extracellulaire, des inhibiteurs de l'ECA, des désactivateurs des radicaux libres, des chélateurs, des antioxydants, des antipolymérases, des agents antiviraux, des agents de thérapie photodynamique, et des agents de thérapie génique.

19. Barrière selon la revendication 1, dans laquelle :
a) les copolymères blocs sont choisis parmi les copolymères blocs d'oxyde d'éthylène et d'oxyde de propylène,
b) les polymères portant des groupes réactifs latents sont choisis parmi les polysaccharides photodérivés, les polyoléfines photodérivés, les polymères de chlorure de vinyle photodérivés, les polymères contenant du fluor photodérivés, les polyacétates de vinyle photodérivés, les alcools polyvinyliques photodérivés, les polyacétals de vinyle photodérivés, les polyacrylates et les polyméthacrylates photodérivés, les polymères et les copolymères de styrène photodérivés, les thermoplastiques vinyle photodérivés, les polyamides et les polyimides photodérivés, les polyacétals photodérivés, les polycarbonates photodérivés, les thermoplastiques contenant des groupes p-phénylene photodérivés, les polyesters photodérivés, les polyuréthanes photodérivés, les polyisocyanurates photodérivés, et les silicones photodérivées
c) le dispositif médical comprend un stent à ballonnet dilatable, et la seconde surface est munie d'un ballonnet dilatable contenu à l'intérieur du stent,
d) l'agent bioactif à l'intérieur des revêtements polymères est présent à une concentration d'au moins 20 % par rapport au poids de la composition revêtue,
e) l'agent bioactif est choisi dans le groupe constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, d'antagonistes de l'hormone de croissance, des facteurs de croissance, des agonistes de la dopamine, des agents radiothérapiques, des peptides, des protéines, des enzymes, des composants de la matrice extracellulaire, des inhibiteurs de l'ECA, des désactivateurs des radicaux libres, des chélateurs, des antioxydants, d'antipolymérases, des agents antiviraux, des agents de thérapie photodynamique, et des agents de thérapie génique, et
f) le revêtement polymère contenant un agent bioactif comprend une pluralité de polymères, comprenant un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe se composant de poly(éthylène-co-vinyle acétate), dans lequel les polyalkyl(méth)acrylates comprennent le poly(n-butyl méthacrylate) et les polyaryl(méth)acrylates sont choisis parmi le poly-9-anthracénylméthacrylate, le polychlorophénylacrylate, le polyméthacryloxy-2-hydroxybenzophénone, le polyméthacryloxybenzotriazole, le polynaphtylacrylate, le polynaphtylméthacrylate, le poly-4-nitrophénylacrylate, les polypentachloro(bromo,fluoro)acrylate et méthacrylate, les polyphénylacrylate et méthacrylate, les polyaralkyl(méth)acrylates sont choisis parmi les polybenzylacrylate et méthacrylate, les poly-2-phénéthylacrylate et méthacrylate, le poly-1-pyrénylméthylméthacrylate, et les polyaryloxyalkyl(méth)acrylates sont choisis parmi les polyphénoxyéthylacrylate et méthacrylate, les polyéthylène glycol phényl éther acrylates et méthacrylates ayant différentes masses moléculaires de polyéthylène glycol.

20. Combinaison comprenant :
a) un dispositif médical implantable ayant une première surface portant un revêtement polymère, contenant un agent bioactif,
b) une seconde surface fournie par un autre matériau positionné en apposition par rapport au dispositif médical, et
c) une barrière positionnée entre la première surface et la seconde surface, la barrière étant choisie dans le groupe constitué par des copolymères blocs et des polymères portant des groupes réactifs latents, dans laquelle la barrière empêche que se produise(nt) des dommages et/ou un délaminage du revêtement polymère, contenant un agent bioactif.

21. Combinaison selon la revendication 20 dans laquelle la barrière est fournie sous la forme d'un revêtement sur le revêtement polymère, d'un revêtement sur la seconde surface, et/ou d'une couche discrète positionnée entre les deux.

22. Combinaison selon la revendication 20 dans laquelle la barrière se compose elle-même d'une ou de plusieurs couches des mêmes matériaux ou de matériaux différents, et positionnée(s) en n'importe quelle combinaison adéquate sur la première et/ou la seconde surface(s), ou fournie(s) séparément entre les deux.

23. Combinaison selon la revendication 20 dans laquelle la barrière est appliquée au cours de la fabrication, du stockage, de la livraison ou du déploiement, et/ou du séjour du dispositif dans le corps.

24. Combinaison selon la revendication 20 dans laquelle le revêtement polymère contenant un agent bioactif est positionné sur la surface d'un dispositif médical implantable, la seconde surface est fournie par la surface d'un matériau différent en apposition par rapport au dispositif, et la barrière comprend une barrière sous la forme d'un revêtement anti-adhérent adapté pour faciliter la mise en place de la surface du dispositif médical et du matériau différent en une apposition stable et séparable l'un par rapport à l'autre.

25. Combinaison selon la revendication 24 dans laquelle le dispositif médical comprend un stent à ballonnet dilatable, et le matériau différent se présente sous la forme d'un ballonnet dilatable à l'intérieur du stent, et la barrière est choisie dans le groupe se composant de copolymères blocs et de polymères portant des groupes réactifs latents.

26. Combinaison selon la revendication 20, dans laquelle :
a) le revêtement polymère contenant un agent bioactif comprend une pluralité de polymères,
b) les copolymères blocs sont choisis parmi les copolymères blocs d'oxyde d'éthylène et d'oxyde de propylène,
c) les polymères portant des groupes réactifs latents sont choisis parmi les polysaccharides photodérivés et les polyoléfines photodérivées, les polymères de chlorure de vinyle photodérivés, les polymères contenant du fluor photodérivés, les polyacétates de vinyle photodérivés, les alcools polyvinyliques photodérivés, les polyacétals de vinyle photodérivés, les polyacrylates et les polyméthacrylates photodérivés, les polymères et les copolymères de styrène photodérivés, les thermoplastiques vinyle photodérivés, les polyamides et les polyimides photodérivés, les polyacétals photodérivés, les polycarbonates photodérivés, les thermoplastiques contenant des groupes p-phénylene photodérivés, les polyesters photodérivés, les polyuréthanes photodérivés, les polyisocyanurates photodérivés, et les silicones photodérivées,
d) le dispositif médical comprend un dispositif médical implantable,
e) l'agent bioactif à l'intérieur des revêtements polymères est présent à une concentration d'au moins 20 % par rapport au poids de la composition revêtue, et
f) des agent bioactif est choisi dans le groupe constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, des antagonistes de l'hormone de croissance, des facteurs de croissance, des agonistes de la dopamine, des agents radiothérapiques, des peptides, des protéines, des enzymes, des composants de la matrice extracellulaire, des inhibiteurs de l'ECA, desdésactivateurs des radicaux libres, des chélateurs, des antioxydants, des antipolymérases, des agents antiviraux, des agents de thérapie photodynamique, et des agents de thérapie génique.

27. Composition selon la revendication 26 dans laquelle :
a) le revêtement polymère contenant un agent bioactif comprend une pluralité de polymères comprenant un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe se composant de poly(éthylène-co-vinyle acétate),
b) les copolymères blocs sont choisis parmi les copolymères blocs d'oxyde d'éthylène et d'oxyde de propylène,
c) les polymères portant des groupes réactifs latents sont choisis parmi l'héparine photodérivée, les polyamides photodérivés et les thermoplastiques vinyle photodérivés,
d) le dispositif médical est choisi dans le groupe constitué par des dispositifs vasculaires, des dispositifs orthopédiques, des dispositifs dentaires, des dispositifs d'administration de produits médicamenteux, des dispositifs ophtalmiques, des dispositifs urologiques, et des prothèses synthétiques,
e) l'agent bioactif à l'intérieur des revêtements polymères est présent à une concentration d'au moins 20 % par rapport au poids de la composition revêtue, et
f) l'agent bioactif est choisi dans le groupe constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, des antagonistes de l'hormone de croissance, des facteurs de croissance, des agonistes de la dopamine, des agents radiothérapiques, des peptides, des protéines, des enzymes, de composants de la matrice extracellulaire, des inhibiteurs de l'ECA, des désactivateurs des radicaux libres, de chélateurs, des antioxydants, des antipolymérases, des agents antiviraux, des agents de thérapie photodynamique, et des agents de thérapie génique.

28. Composition selon la revendication 20 dans laquelle :
a) le revêtement polymère contenant un agent bioactif comprend une pluralité de polymères comprenant un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe est constitué par poly(éthylène-co-vinyle acétate),
b) les copolymères blocs sont choisis parmi les copolymères blocs d'oxyde d'éthylène et d'oxyde de propylène,
c) les polymères portant des groupes réactifs latents comprennent une héparine photodérivée, un polyacrylamide photodérivé, ou une polyvinylpyrrolidone photodérivée,
d) le dispositif médical comprend un dispositif médical implantable choisi dans le groupe constitué par des dispositifs vasculaires, des dispositifs orthopédiques, des dispositifs dentaires, des dispositifs d'administration de produits médicamenteux, des dispositifs ophtalmiques, des dispositifs urologiques, et des prothèses synthétiques, et la seconde surface est fournie par une autre partie de contact du même dispositif ou en tant que matériau différent contenu à l'intérieur ou autour du dispositif.
e) l'agent bioactif à l'intérieur des revêtements polymères est présent à une concentration d'au moins 20 % par rapport au poids de la composition de revêtue, et
f) l'agent bioactif est choisi dans le groupe est constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, des antagonistes de l'hormone de croissance, de facteurs de croissance, des agonistes de la dopamine, des agents radiothérapiques, des peptides, des protéines, des enzymes, de composants de la matrice extracellulaire, des inhibiteurs de l'ECA, des désactivateurs des radicaux libres, des chélateurs, des antioxydants, des antipolymérases, des agents antiviraux, des agents de thérapie photodynamique, et des agents de thérapie génique.

29. Combinaison selon la revendication 20, dans laquelle :
a) les copolymères blocs sont choisis parmi les copolymères blocs d'oxyde d'éthylène et d'oxyde de propylène,
b) les polymères portant des groupes réactifs latents sont choisis parmi les polysaccharides photodérivés, les polyoléfines photodérivés, les polymères de chlorure de vinyle photodérivés, les polymères contenant du fluor photodérivés, les polyacétates de vinyle photodérivés, les alcools polyvinyliques photodérivés, les polyacétals de vinyle photodérivés, les polyacrylates et les polyméthacrylates photodérivés, les polymères et les copolymères de styrène photodérivés, les thermoplastiques vinyle photodérivés, les polyamides et les polyimides photodérivés, les polyacétals photodérivés, les polycarbonates photodérivés, les thermoplastiques contenant des groupes p-phénylene photodérivés, les polyesters photodérivés, les polyuréthanes photodérivés, les polyisocyanurates photodérivés, et les silicones photodérivées,
c) le dispositif médical comprend un stent à ballonnet dilatable, et la seconde surface est munie d'un ballonnet dilatable contenu à l'intérieur du stent,
d) l'agent bioactif à l'intérieur des revêtements polymères est présent à une concentration d'au moins 20 % par rapport au poids de la composition revêtue,
e) l'agent bioactif est choisi dans le groupe constitué par des inhibiteurs de thrombine, des agents antithrombogéniques, des agents thrombolytiques, des agents fibrinolytiques, des inhibiteurs du vasospasme, des bloqueurs du canal calcique, des vasodilatateurs, des agents antihypertenseurs, des agents antimicrobiens, des antibiotiques, des inhibiteurs des récepteurs glycoprotéiques de surface, des agents antiplaquettaires, des antimitotiques, des inhibiteurs des microtubules, des agents anti-secréteurs, des inhibiteurs d'actine, des inhibiteurs remodelants, des nucléotides antisens, des anti métabolites, des antiproliférants (y compris les agents anti-angiogéniques), des agents chimiothérapiques anticancéreux, des agents anti-inflammatoires stéroïdiens ou non stéroïdiens, des agents immunosuppresseurs, d'antagonistes de l'hormone de croissance, des facteurs de croissance, des agonistes de la dopamine, des agents radiothérapiques, des peptides, des protéines, des enzymes, des composants de la matrice extracellulaire, des inhibiteurs de l'ECA, des désactivateurs des radicaux libres, des chélateurs, des antioxydants, des antipolymérases, des agents antiviraux, des agents de thérapie photodynamique, et des agents de thérapie génique, et
f) le revêtement polymère contenant un agent bioactif comprend une pluralité de polymères, comprenant un premier polymère choisi dans le groupe constitué par des polyalkyl(méth)acrylates, des polyaryl(méth)acrylates, des polyaralkyl(méth)acrylates, et des polyaryloxyalkyl(méth)acrylates, et un deuxième polymère choisi dans le groupe est constitué par du poly(éthylène-co-vinyle acétate), dans lequel les polyalkyl(méth)acrylates comprennent le poly(n-butyl méthacrylate) et les polyaryl(méth)acrylates sont choisis parmi le poly-9-anthracénylméthacrylate, le polychlorophénylacrylate, le polyméthacryloxy-2-hydroxybenzophénone, le polyméthacryloxybenzotriazole, le polynaphtylacrylate, le polynaphtylméthacrylate, le poly-4-nitrophénylacrylate, les polypentachloro(bromo,fluoro)acrylate et méthacrylate, les polyphénylacrylate et méthacrylate, les polyaralkyl(méth)acrylates sont choisis parmi les polybenzylacrylate et méthacrylate, les poly-2-phénéthylacrylate et méthacrylate, le poly-1-pyrénylméthylméthacrylate, et les polyaryloxyalkyl(méth)acrylates sont choisis parmi les polyphénoxyéthylacrylate et méthacrylate, les polyéthylène glycol phényl éther acrylates et méthacrylates ayant différentes masses moléculaires de polyéthylène glycol.

30. Utilisation d'une barrière selon la revendication 1 destinée à minimiser les dommages produits sur une première surface comprenant une composition polymère contenant un agent bioactif sur une surface d'un dispositif médical, par une seconde surface fournie par un autre matériau et positionnée en apposition par rapport à la première surface.
